# EUROPEAN PATENT APPLICATION

(11) **EP 4 104 816 A1**
(43) Date of publication of application: **21.12.2022**
(21) Application number: 21179203.1
(22) Date of filing: 14.06.2021
(51) Int. Cl.: A61K 9/00, A61K 9/70, A61K 47/02, A61K 47/10

(54) **NON-NANOPARTICULATE APPLICATIONS FORMS OF MACROLIDES**

(71) Applicant: Nucleus Medical GmbH, 82031 Grünwald (DE)
(72) Inventor: HORSTKOTTE, Elke, 82031 Grünwald (DE); BEIER, Wolfgang, 82031 Grünwald (DE)
(74) Representative: Engelhard, Markus

(57) **Abstract**

The present invention relates to a mucoadhesive layer, preferably mucoadhesive buccal layer, comprising a non-nanoparticulate macrolide. Furthermore, the present invention relates to a mucoadhesive film, preferably mucoadhesive buccal film, comprising a mucoadhesive layer comprising a non-nanoparticulate macrolide, and further comprising a backing layer. The present invention also relates to a mucoadhesive film, preferably mucoadhesive buccal film, comprising a mucoadhesive layer, a backing layer, and an intermediate layer. The present invention further relates to a mucoadhesive layer or mucoadhesive film for use as a medicament and for use in preventing and/or treating transplant rejection. Moreover, the present invention relates to a method of preparing a mucoadhesive film.

## Description

### FIELD OF THE INVENTION

The present invention relates to a mucoadhesive layer, preferably mucoadhesive buccal layer, comprising a non-nanoparticulate macrolide. Furthermore, the present invention relates to a mucoadhesive film, preferably mucoadhesive buccal film, comprising a mucoadhesive layer comprising a non-nanoparticulate macrolide, and further comprising a backing layer. The present invention also relates to a mucoadhesive film, preferably mucoadhesive buccal film, comprising a mucoadhesive layer, a backing layer, and an intermediate layer. The present invention further relates to a mucoadhesive layer or mucoadhesive film for use as a medicament and for use in preventing and/or treating transplant rejection. Moreover, the present invention relates to a method of preparing a mucoadhesive film.

### BACKGROUND OF THE INVENTION

Poor water solubility of active agents has been a challenge in the development of pharmaceutical formulations. In fact, the majority of active pharmaceutical ingredients exhibit a low water solubility. To evoke a therapeutic effect, the active agent has to dissolve and reach the bloodstream in sufficient concentration. Accordingly, active agents with low water solubility, such as macrolides, typically have poor bioavailability, i.e. only a small proportion of the administered active agent reaches the bloodstream after ingestion. The low bioavailability of the drugs reflects incomplete absorption in the gastrointestinal tract and/or metabolism in the gastrointestinal tract. The rate and extent of absorption of macrolides, such as tacrolimus, may even be reduced in the presence of food.

Macrolides such as tacrolimus, which has a narrow therapeutic index, exhibit poor oral bioavailability due to such poor solubility, and further show high inter- and intra-individual variability in the pharmacokinetic parameters. Therefore, the doses administered to a patient have to be carefully monitored. Due to the patient-specific dosing of the macrolide tacrolimus (mg/kg body weight/day), the available capsule preparations are often opened, the active ingredient is brought into solution and then applied to the patient; such process being called "drug compounding". Drug compounding does not meet the high quality requirements of a pharmaceutical product, and may even jeopardize drug safety by increasing the risk of incorrect dosing. Furthermore, drug compounding may result in reduced stability of the drug or microbial contamination of the prepared solutions.

Drug delivery via the buccal mucosa represents a route of administration providing direct access to the systemic circulation and bypassing hepatic first pass metabolism. Buccal drug delivery applications require drugs with appropriate water solubility due to permeation requirements through the buccal mucosa and systemic drug absorption. A mucoadhesive buccal film has been proposed as a therapeutic system for providing the macrolide tacrolimus. Tacrolimus is classified as BCS class II drug with a low solubility and high permeability and reveals a high melting point at 126°C. Due to the fact that tacrolimus is a narrow therapeutic index drug, stricter bioequivalence criteria are required for generic tacrolimus products, as small changes in dose or exposure can result in therapeutic failure or toxicity. EP 3 662 894 A1 relates to nanoparticles comprising tacrolimus and to a mucoadhesive buccal film containing such nanoparticles. The production of such buccal film requires the preparation of tacrolimus nanoparticles. There remains the need for mucoadhesive buccal films that allow to effectively deliver macrolides to a patient. Particularly, there remains the need for enhanced formulations of macrolides which allow to achieve sufficient blood concentrations of macrolide. Furthermore, there remains the need for efficient methods of preparing buccal films comprising tacrolimus.

Thus, there remains the need for providing formulations of macrolides with increased efficacy and/or enhanced bioavailability. Particularly, formulations that allow to efficiently administer sufficient doses of macrolides to a patient are needed. Furthermore, there is the need for enhanced means such as administration forms for administering macrolides, e.g. therapeutic systems that allow an efficient administration of active agents such as macrolides.

### SUMMARY OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to a mucoadhesive layer, preferably mucoadhesive buccal layer, comprising a non-nanoparticulate macrolide.

In one embodiment, the non-nanoparticulate macrolide is selected from macrolide immune suppressants, macrolide antibiotics, and macrolide antifungals;
wherein, preferably,
- the macrolide immune suppressant is selected from tacrolimus, sirolimus, everolimus, and pimecrolimus;
- the macrolide antibiotic is selected from erythromycin, clarithromycin, azithromycin, roxithromycin, josamycin, spiramycin, telithromycin, tylosin, and fidaxomicin; and/or
- the macrolide antifungal is selected from polyenes, in particular nystatin, natamycin, and amphotericin B,
wherein, more preferably, said non-nanoparticulate macrolide is non-nanoparticulate tacrolimus or sirolimus, even more preferably non-nanoparticulate tacrolimus.

In one embodiment, said non-nanoparticulate macrolide is present in said layer in the form of micronized macrolide, preferably micronized crystalline macrolide, and/or in a molecularly dissolved non-nanoparticulate form.

In one embodiment, said micronized macrolide has an average particle size of from 1 to 100 µm, preferably of from 1.5 to 50 µm, more preferably of from 2 to 25 µm.

In one embodiment, the mucoadhesive layer comprises
- a mucoadhesive polymer, preferably a polymer selected from a) amphiphilic polymers and hydrophilic polymers, b) poly(methacrylates), c) crosslinked polyacrylic acid polymers, d) copolymers of methyl vinyl ether and maleic anhydride, e) polymers comprising polyvinyl acetate and/or polyvinylpyrrolidone, and combinations thereof;
   wherein, preferably,
   the amphiphilic polymers are selected from polyvinyl caprolactam-polyvinyl acetate-polyethylene glycol graft copolymers, polyoxyl castor oils and D-α-tocopherol-polyethylenglycol-succinate (TPGS), and/or
   the poly(methacrylates) are selected from neutral protonizable poly(methacrylates) and cationic poly(methacrylates), preferably selected from copolymers of dialkylaminoethyl methacrylates and methacrylic acid ester(s), and copolymers of trialkylammonioethyl methacrylates and methacrylic acid ester(s), in particular copolymers of dimethylaminoethyl methacrylates and methacrylic acid esters, and copolymers of trimethylammonioethyl methacrylates and methacrylic acid esters;
and wherein the mucoadhesive layer further comprises:
- a cellulose derivative, preferably selected from hydroxypropyl cellulose (HPC), carboxymethyl cellulose (CMC), hydroxypropyl methylcellulose (HPMC), hydroxyethyl cellulose (HEC), methyl cellulose (MC), hydroxyethyl methyl cellulose (HEMC), ethyl cellulose (EC), and a combination thereof;
- a plasticizer; preferably selected from glycerol, polyethylene glycol, e.g. low molecular weight polyethylene glycol such as PEG 200 or PEG 400, propylene glycol, sorbitol, and tributylcitrate; more preferably glycerol;
- optionally a colorant, preferably TiO₂;
- and the non-nanoparticulate macrolide.

In one embodiment, said non-nanoparticulate macrolide is present in said layer in the molecularly dissolved non-nanoparticulate form;
and said mucoadhesive polymer and/or said cellulose derivative is a polymer soluble in an organic solvent.

In one embodiment, the mucoadhesive layer comprises
a) a mucoadhesive polymer, preferably an amphiphilic polymer; hydroxypropyl cellulose (HPC); carboxymethyl cellulose (CMC); a plasticizer, preferably glycerol; and optionally a colorant, preferably TiO₂;
   preferably 10 wt% - 30 wt% of mucoadhesive polymer; 25 wt% - 35 wt% of hydroxypropyl cellulose (HPC); 10 wt% - 25 wt% of carboxymethyl cellulose (CMC); 10 wt% - 16 wt% of said plasticizer; and optionally 1 wt% - 4 wt% of said colorant; or
b) a mucoadhesive polymer, preferably selected from crosslinked polyacrylic acid polymers and copolymers of methyl vinyl ether and maleic anhydride; hydroxypropyl cellulose (HPC); ethyl cellulose (EC); a plasticizer, preferably glycerol; and optionally a colorant, preferably TiO₂;
   preferably 2 wt% - 17 wt% of mucoadhesive polymer; 30 wt% - 70 wt% of hydroxypropyl cellulose (HPC); 5 wt% - 40 wt% of said ethyl cellulose; 3 wt% - 20 wt% of said plasticizer; and optionally 1 wt% - 5 wt% of said colorant.

In one embodiment, said macrolide is present in said mucoadhesive layer at a concentration of at least 2 wt%, preferably at least 3 wt%, e.g. 3.2 wt%; and/or in an amount in the range of from approximately 0.01 mg to approximately 10 mg, with reference to 1 cm² of said mucoadhesive layer, preferably in an amount in the range of from approximately 0.1 mg to approximately 5 mg, with reference to 1 cm² of said mucoadhesive layer, more preferably in the range of from approximately 1.5 mg to approximately 3.5 mg, with reference to 1 cm² of said mucoadhesive layer.

In a further aspect, the present invention relates to a mucoadhesive film, preferably mucoadhesive buccal film, comprising a mucoadhesive layer comprising a non-nanoparticulate macrolide, preferably a mucoadhesive layer as defined above; and further comprising a backing layer, preferably a backing layer impermeable for the macrolide and/or impermeable for water.

In one embodiment, the backing layer comprises a plasticizer, preferably selected from glycerol, polyethylene glycol, e.g. low molecular weight polyethylene glycol such as PEG 200 or PEG 400, propylene glycol, sorbitol, and tributylcitrate, more preferably glycerol; and a water-insoluble polymer and/or a cellulose derivative, e.g. hydroxyethyl cellulose, preferably a water-insoluble cellulose derivative, more preferably ethyl cellulose.

In one embodiment, the mucoadhesive film further comprises an intermediate layer arranged between the mucoadhesive layer and the backing layer;
wherein, preferably, said intermediate layer facilitates adhesion between the mucoadhesive layer and the backing layer;
wherein, optionally, said intermediate layer comprises a plasticizer, preferably selected from glycerol, polyethylene glycol, e.g. low molecular weight polyethylene glycol such as PEG 200 or PEG 400, propylene glycol, sorbitol, and tributylcitrate, more preferably glycerol; and a polymer, preferably a polymer comprising polyvinyl acetate and/or polyvinylpyrrolidone, more preferably a vinylpyrrolidone-vinyl acetate copolymer.

In one embodiment, said mucoadhesive layer has an area weight in the range of from 70 to 180 g/m², preferably in the range of from 100 to 150 g/m²; and/or
said backing layer has an area weight in the range of from 40 to 100 g/m², preferably in the range of from 40 to 60 g/m², e.g. 50 g/m²; and/or
said intermediate layer, if present, has an area weight in the range of from 40 to 100 g/m², preferably in the range of from 40 to 70 g/m², e.g. 60 g/m².

In one embodiment, said mucoadhesive layer and/or backing layer comprise(s) a colorant;
wherein, preferably, said mucoadhesive layer and said backing layer each comprise a colorant, wherein said mucoadhesive layer comprises a first colorant, e.g. TiO₂, and said backing layer comprises a second colorant, e.g. brilliant blue, wherein said first colorant and said second colorant are different from each other.

In one embodiment, the mucoadhesive layer has a water content of < 5 wt%, preferably < 3 wt%, more preferably < 2 wt%.

In one embodiment, said non-nanoparticulate macrolide and/or said mucoadhesive layer are as defined above.

In a further aspect, the present invention relates to a mucoadhesive film, preferably mucoadhesive buccal film, comprising a mucoadhesive layer, a backing layer, and an intermediate layer,
wherein said intermediate layer is arranged between the mucoadhesive layer and the backing layer, and wherein said mucoadhesive layer comprises a macrolide, preferably tacrolimus; wherein, preferably, said intermediate layer facilitates adhesion between the mucoadhesive layer and the backing layer;
wherein, optionally, said intermediate layer comprises a plasticizer, preferably selected from glycerol, polyethylene glycol, e.g. low molecular weight polyethylene glycol such as PEG 200 or PEG 400, propylene glycol, sorbitol, and tributylcitrate, more preferably glycerol; wherein, optionally, said intermediate layer comprises a polymer, preferably a polymer comprising polyvinyl acetate and/or polyvinylpyrrolidone, more preferably a vinylpyrrolidone-vinyl acetate copolymer.

In one embodiment, said mucoadhesive layer, said backing layer, and/or said intermediate layer are as defined above.

In a further aspect, the present invention relates to a mucoadhesive layer, as defined above, or a mucoadhesive film, as defined above, for use as a medicament.

In a further aspect, the present invention relates to a mucoadhesive layer, as defined above, or a mucoadhesive film, as defined above, for use in preventing and/or treating transplant rejection, preferably solid organ transplant rejection such as liver, kidney or heart allograft rejection;
wherein, optionally, said use involves an attachment of said film or layer in the body cavity of a patient, preferably in a buccal cavity of a patient.

In a further aspect, the present invention relates to a method of preparing a mucoadhesive film, comprising
i) providing a micronized macrolide, preferably non-nanoparticulate micronized macrolide; and a polymer mixture, wherein said polymer mixture comprises
   - a mucoadhesive polymer, preferably a polymer selected from a) amphiphilic polymers and hydrophilic polymers, b) poly(methacrylates), c) crosslinked polyacrylic acid polymers, d) copolymers of methyl vinyl ether and maleic anhydride, e) polymers comprising polyvinyl acetate and/or polyvinylpyrrolidone, and combinations thereof;
   - a cellulose derivative, preferably selected from hydroxypropyl cellulose (HPC), carboxymethyl cellulose (CMC), hydroxypropyl methylcellulose (HPMC), hydroxyethyl cellulose (HEC), methyl cellulose (MC), hydroxyethyl methyl cellulose (HEMC), ethyl cellulose (EC), and a combination thereof;
   - optionally, a plasticizer; preferably selected from glycerol, polyethylene glycol, e.g. low molecular weight polyethylene glycol such as PEG 200 or PEG 400, propylene glycol, sorbitol, and tributylcitrate; more preferably glycerol;
   - optionally a colorant, preferably TiO₂;
ii) optionally, dispersing the micronized macrolide in a liquid medium to obtain a micronized macrolide dispersion and/or dissolving said micronized macrolide in an organic solvent to obtain a micronized macrolide solution;
iii) mixing the micronized macrolide, optionally micronized macrolide dispersion or micronized macrolide solution, with the polymer mixture to obtain a mucoadhesive layer composition;
iv) preparing a mucoadhesive layer using the mucoadhesive layer composition; optionally by coating the mucoadhesive layer composition on a surface, preferably on a process liner, e.g. by coating, hot melt extrusion, and/or casting;
v) optionally, drying said mucoadhesive layer, thereby obtaining the mucoadhesive film comprising said mucoadhesive layer.

In one embodiment, said method further comprises preparing a backing layer, comprising
a) providing a backing layer polymer mixture; optionally comprising a plasticizer, preferably glycerol, and a water-insoluble polymer, preferably a water-insoluble cellulose derivative, more preferably ethyl cellulose;
b) preparing a backing layer using the backing layer polymer mixture, optionally on a surface of said mucoadhesive layer, e.g. by coating said polymer mixture on a surface of said mucoadhesive layer;
c) optionally, drying the backing layer.

In one embodiment, said method further comprises preparing an intermediate layer, comprising
1) providing an intermediate layer polymer mixture; optionally comprising a plasticizer, preferably glycerol, and a polymer, preferably a polymer comprising polyvinyl acetate and/or polyvinylpyrrolidone, more preferably a vinylpyrrolidone-vinyl acetate copolymer;
2) preparing an intermediate layer using the polymer mixture, optionally on a surface of said mucoadhesive layer, e.g. by coating said polymer mixture on a surface of said mucoadhesive layer;
3) optionally, drying the intermediate layer.

In one embodiment, said method further comprises a step of joining said mucoadhesive layer and said backing layer, optionally by compression and/or lamination, e.g. thermo-lamination.

In one embodiment, said method further comprises a step of joining said mucoadhesive layer, said intermediate layer, and said backing layer, optionally by compression and/or lamination, e.g. thermo-lamination.

In one embodiment, said macrolide, said non-nanoparticulate micronized macrolide, said mucoadhesive polymer, said plasticizer, mucoadhesive layer, said backing layer, and/or said intermediate layer are as defined above.

In a further aspect, the present invention relates to a method of preventing and/or treating transplant rejection, preferably solid organ transplant rejection such as liver, kidney or heart allograft rejection, comprising administering a mucoadhesive layer, as defined above, or a mucoadhesive film, as defined above, to a patient in need thereof.

In one embodiment, said administering involves an attachment of said film or layer in the body cavity of a patient, preferably in a buccal cavity of a patient.

In one embodiment, said mucoadhesive layer or mucoadhesive film comprises a therapeutically effective amount of a non-nanoparticulate macrolide.

In one embodiment, the mucoadhesive layer, the mucoadhesive film, and/or the non-nanoparticulate macrolide are as defined above.

In a further aspect, the present invention relates to a use of a non-nanoparticulate macrolide for the manufacture of a mucoadhesive layer and/or mucoadhesive film, preferably for preventing and/or treating transplant rejection.

In one embodiment, the mucoadhesive layer, the mucoadhesive film, the non-nanoparticulate macrolide, and/or the preventing and/or treating transplant rejection are as defined above.

### DETAILED DESCRIPTION

The present invention aims at providing a mucoadhesive film for application of macrolides, for example for preventing and/or treating transplant rejection after transplantations of organs, particularly solid organ transplantations. Furthermore, the present invention aims at providing a unidirectional mucoadhesive film, i.e. a film which releases the macrolide directly into the mucosa and with negligible release to the cavity of a body cavity, e.g. buccal cavity. The present invention further aims at providing an efficient method of preparing a mucoadhesive film. The present invention further aims at providing a mucoadhesive film which can be efficiently prepared. Furthermore, it is an aim of the invention to prepare a mucoadhesive film which prevents swallowing of a macrolide. It is also an aim of the invention to provide a therapeutic system, particularly a mucoadhesive film, that allows to achieve therapeutic blood levels of a macrolide. Moreover, it is an aim of the invention to provide a therapeutic system, particularly a mucoadhesive film, that allows for an early onset of a therapeutic effect.

Although macrolides such as tacrolimus have a very low water solubility, the layer and film of the invention surprisingly achieve therapeutic macrolide blood concentrations. Unexpectedly, clinically relevant blood levels of macrolides are achieved with a layer and film of the invention. Moreover, surprisingly, a film and layer of the invention comprising crystalline or molecularly dissolved non-nanoparticulate macrolide is highly stabile against degradation.

The term "mucoadhesive layer", as used herein, relates to a layer that adheres to a mucosa and/or that is configured to adhere to a mucosa, e.g. the oral and/or buccal mucosa. In one embodiment, the mucoadhesive layer comprises a mucoadhesive polymer and/or a cellulose derivative, and further comprises non-nanoparticulate macrolide. In one embodiment, the mucoadhesive layer comprises non-nanoparticulate macrolide, preferably micronized crystalline non-nanoparticulate macrolide and/or molecularly dissolved non-nanoparticulate macrolide. In one embodiment, the mucoadhesive layer optionally comprises a surfactant, e.g. polysorbate. In one embodiment, the mucoadhesive layer is swellable in an aqueous liquid, but is insoluble in the aqueous liquid and/or has negligible solubility in the aqueous liquid. The insolubility or low solubility increases the adhesive time on the mucosa and thus enables that the active ingredient is released over a long period of time, e.g. for at least 60 min. The term "mucoadhesive buccal layer" or "MBF", as used herein, relates to a mucoadhesive layer which is configured for a buccal application.

The term "macrolide", as used herein, relates to a class of natural products that consist of a large macrocyclic lactone ring, e.g. a 14-, 15-, or 16-membered lactone ring, to which one or more deoxy sugars, usually cladinose and desosamine, may be attached; e.g. tacrolimus, sirolimus, everolimus, pimecrolimus, erythromycin, clarithromycin, azithromycin, roxithromycin, josamycin, spiramycin, telithromycin, tylosin, and fidaxomicin, nystatin, natamycin, and amphotericin B. In one embodiment, the macrolide is selected from macrolide immune suppressants, macrolide antibiotics, and macrolide antifungals. In one embodiment, the macrolide immune suppressant is selected from tacrolimus, sirolimus, everolimus, and pimecrolimus. In one embodiment, the macrolide antibiotic is selected from erythromycin, clarithromycin, azithromycin, roxithromycin, josamycin, spiramycin, telithromycin, tylosin, and fidaxomicin. In one embodiment, the macrolide antifungal is selected from polyenes, in particular nystatin, natamycin, and amphotericin B. In a preferred embodiment, the macrolide is selected from tacrolimus, sirolimus, everolimus, and pimecrolimus, preferably tacrolimus and sirolimus, more preferably tacrolimus. In one embodiment, the terms "active agent", "active ingredient", and "API" relate to the macrolide.

The term "non-nanoparticulate macrolide", as used herein, relates to the macrolide not being in the form of nanoparticles. In one embodiment, non-nanoparticulate macrolide relates to macrolide that is not formulated as nanoparticles, particularly not formulated as nanoparticles having a size in the range of 1 nm to 1000 nanometers. In one embodiment, non-nanoparticulate macrolide is macrolide which is formulated as molecularly dissolved macrolide and/or micronized crystalline macrolide. In one embodiment, molecularly dissolved macrolide is not molecularly dissolved macrolide in the form of nanoparticles, e.g. is not molecularly dissolved macrolide present in a micelle or liposome. In one embodiment, molecularly dissolved macrolide does not relate to nanoparticles comprising molecularly dissolved macrolide. In one embodiment, molecularly dissolved macrolide is formulated in the form of a solid solution, e.g. embedded and/or dissolved in a polymer matrix. In one embodiment, micronized crystalline macrolide is not in the form of nanoparticles, e.g. is not in the form of nanoparticles comprising micronized crystalline macrolide. In one embodiment, micronized crystalline macrolide has an average particle size of > 1000 nm. In one embodiment, micronized crystalline macrolide is in the form of microparticles, preferably having an average particle size, in the range of 1 µm 1000 µm, preferably > 1000 nm. In one embodiment, non-nanoparticulate macrolide comprised by a mucoadhesive layer and/or mucoadhesive film of the invention is not in the form of nanoparticles and/or is not comprised by nanoparticles. In one embodiment, said non-nanoparticulate macrolide is present in said layer in the form of micronized macrolide, preferably micronized crystalline macrolide, in a molecularly dissolved non-nanoparticulate form, and/or in the form of crystalline macrolide; preferably said non-nanoparticulate macrolide is present in said layer in the form of micronized macrolide, preferably micronized crystalline macrolide, and/or in a molecularly dissolved non-nanoparticulate form. In one embodiment, a mucoadhesive layer comprising macrolide in the form of micronized macrolide, preferably micronized crystalline macrolide, and/or in a molecularly dissolved non-nanoparticulate form, further comprises macrolide in a crystalline form.

The term "average particle size", as used herein, relates to the average diameter of an analyzed batch of particles, e.g. (micro)particles of micronized macrolide. For example, the average particle size may be the average diameter of a batch of particles, wherein, if the particles are not spherical, the longest extension of each particle is considered as the diameter. In one embodiment, the average particle size is in the range of from 1 to 100 µm, preferably of from 1.5 to 50 µm, more preferably of from 2 to 25 µm. In one embodiment, an average particle size, e.g. of a micronized macrolide, is measured using any technique known to a person skilled in the art, for example laser diffraction analysis or dynamic light scattering, preferably laser diffraction analysis. The average particle size is defined as the average diameter of the analyzed particles determined by a suitable process, e.g. using dynamic light scattering. In one embodiment, the average particle size of a micronized macrolide is 2 µm to 25 µm. In one embodiment, the micronized macrolide has a particle size distribution D₁₀ 2 µm; D₅₀ 10 µm; and D₉₀ 25 µm. In one embodiment, 10% of all particles had a particle size <2 µm, 50% of all particles had a particle size <10 µm, and 90% of all particles had a particle size <10 µm. In one embodiment, all particles had a particle size > 1 µm. In one embodiment, the micronized macrolide is not in the form of nanoparticles. In one embodiment, the average particle size is D₅₀. In one embodiment, the average particle size of micronized macrolide is > 1 µm, it being understood that, preferably, none of the particles is a nanoparticle. In one embodiment, the particle size of micronized macrolide is > 1 µm. In one embodiment, the particle size of each micronized macrolide particle is > 1 µm. The term "micronized", as used herein, relates to the average particle diameters being in the micrometer range and/or being reduced to the micrometer range, preferably by micronization. In one embodiment, "micronized crystalline macrolide" relates to crystalline macrolide having an average particle diameter in the micrometer range.

In one embodiment, the term "mucoadhesive film", as used herein, relates to a film that adheres to a mucosa and/or that is configured to adhere to a mucosa, e.g. the oral and/or buccal mucosa. In one embodiment, the terms "film", "mucoadhesive film", "mucoadhesive buccal film", and "MBF" are used interchangeably. In one embodiment, when referring to "mucoadhesive buccal film" or "MBF", such abbreviation does not only relate to a mucoadhesive buccal film, but also to a mucoadhesive film for other applications than a buccal application. In one embodiment, the mucoadhesive film is a solid preparation comprising a single or multiple layers of suitable material(s) intended to be applied to a body cavity, preferably the buccal cavity, to obtain a systemic effect or a local effect, preferably a systemic effect; and/or the mucoadhesive film is a flexible single-dose preparation configured to be applied to a body cavity, preferably the oral cavity, to obtain either a systemic or a local effect by delivering the macrolide over a certain period of time, after which it is then removed. In one embodiment, the mucoadhesive film is an oromucosal patch.

In one embodiment, the mucoadhesive film comprises a mucoadhesive layer and optionally a backing layer. In a preferred embodiment, the mucoadhesive film comprises a mucoadhesive layer and a backing layer. In one embodiment, the mucoadhesive film comprises a mucoadhesive layer; a backing layer, preferably an impermeable backing layer; and optionally an intermediate layer. In one embodiment, the mucoadhesive layer contains the macrolide in a polymer matrix and provides prolonged contact of the macrolide with the buccal mucosa. In one embodiment, the film comprises at least two layers, for example two layers laminated to each other; preferably a mucoadhesive layer containing the active ingredient and a backing layer. In one embodiment, to prevent a release of the macrolide to the oral cavity, the backside of the mucoadhesive layer is sealed with a backing layer, preferably a water-insoluble and/or macrolide impermeable backing layer. In one embodiment, the backside of the mucoadhesive layer is the side configured to be facing the oral cavity. In one embodiment, the two layers are attached to each other via an intermediate layer, preferably an adhesive layer. In one embodiment, the mucoadhesive layer is configured to be administered to a patient, e.g., by incorporating the mucoadhesive layer in a mucoadhesive film, an oromucosal patch, or any other application system.

In one embodiment, the adhesion characteristic of the mucoadhesive layer and/or film is sufficient for an application of at least 30 min, e.g. at least 60 min, i.e. the layer and/or film adheres to the mucosa for at least 30 min, e.g. at least 60 min. In one embodiment, ethyl cellulose is comprised by a mucoadhesive layer and/or film and allows for an application time of at least 30 min, e.g. at least 60 min due to its low water solubility. In one embodiment, the backing layer comprises ethyl cellulose. In one embodiment, the mucoadhesive film is a non-dissolvable film that must be removed from the mucosa, e.g. buccal mucosa, after drug release. In one embodiment, the mucoadhesive film is applied to a patient for a period of at least 30 min, preferably 30 to 75 min.

In one embodiment, the mucoadhesive layer and/or the mucoadhesive film has/have an area of 0.5 cm² to 10 cm², preferably 2 cm² to 8 cm². In one embodiment, the area weight of the mucoadhesive film is about 20 g/m² to about 300 g/m², preferably about 50 g/m² to about 260 g/m². In one embodiment, the mucoadhesive film has a film thickness of about 20 µm to about 1000 µm, preferably of about 50 µm to about 500 µm, more preferably of about 50 µm to about 300 µm, e.g. about 150 µm to about 260 µm.

In one embodiment, to be able to distinguish between the adhesive layer and the backing layer, at least one of the layers comprises a colorant, e.g. a white pigment is comprised by the mucoadhesive layer and a blue dye is comprised by the backing layer. In one embodiment, the mucoadhesive film further comprises a flavoring agent and/or sweetening agent, such as an agent selected from citric acid, peppermint oil, sodium saccharin, and citrus flavor. In one embodiment, such flavoring agent and/or sweetening agent mask(s) an unpleasant taste of the macrolide or other MBF components. In one embodiment, at least one layer further comprises at least one adjuvant selected from the group comprising stabilizing agents, e.g. chelators, colorants, flavorings, sweeteners, taste masking agents, emulsifiers, enhancers, pH regulators, humectants, preservatives and/or antioxidants. In one embodiment, at least one layer comprises a chelator, preferably EDTA. In one embodiment, when referring to a "layer", the mucoadhesive layer, the intermediate layer, and/or the backing layer are meant.

In one embodiment, the dose strength of the film is determined by the size and/or area of the film, wherein, optionally, the quantitative composition per film area is constant. In one embodiment, the film comprises a dose of up to 10 mg macrolide. In one embodiment, an amount of macrolide is measured using HPLC. In one embodiment, HPLC is used to quantify a macrolide. In one embodiment, the mucoadhesive film is administered and/or configured to be administered to adult patients or pediatric patients, e.g. children of the age up to about 18 years.

In one embodiment, said mucoadhesive layer and/or backing layer comprise(s) a colorant. In one embodiment, said mucoadhesive layer and said backing layer each comprise a colorant, wherein said mucoadhesive layer comprises a first colorant, e.g. TiO₂, and said backing layer comprises a second colorant, e.g. brilliant blue, wherein said first colorant and said second colorant are different from each other. In one embodiment, the mucoadhesive layer and/or the mucoadhesive film has/have a water content of < 5 wt%, preferably < 3 wt%, more preferably < 2 wt%.

The term "plasticizer", as used herein, relates to any plasticizer known to a person skilled in the art, preferably plasticizers which are suitable for in vivo application. In one embodiment, the plasticizer, e.g. a plasticizer comprised by a mucoadhesive layer, an intermediate layer, and/or a backing layer, is selected from glycerol, poly(ethylene glycol), preferably low molecular weight poly(ethylene glycol) such as PEG 200 or PEG 400, propylene glycol, sorbitol, and tributylcitrate. In one embodiment, low molecular weight poly(ethylene glycol) has a molecular weight ≤ 600 daltons, preferably ≤ 450 daltons, e.g. 420 daltons.

The term "backing layer", as used herein, relates to a layer of a mucoadhesive film. In one embodiment, the backing layer covers the backside of a mucoadhesive layer in a mucoadhesive film. The backside is preferably the side of the mucoadhesive layer or film configured to be facing the body cavity, preferably oral cavity, i.e. the side facing away from the mucosa. In one embodiment, the backing layer is impermeable for macrolide and/or impermeable for water. In one embodiment, the backing layer prevents macrolide release from the mucoadhesive layer into a body cavity, e.g. into the oral cavity. In one embodiment, the backing layer covers the backside of the mucoadhesive layer and optionally further covers the side regions of the mucoadhesive layer. In one embodiment, the backing layer is a sealing layer. In one embodiment, the backing layer, preferably impermeable backing layer, prevents undesired release of the macrolide into the body cavity, preferably oral cavity. In one embodiment, the backing layer is configured to ensure unidirectional release of the macrolide into the mucosa, preferably buccal mucosa. In one embodiment, the backing layer prevents release of the macrolide into the buccal cavity. In one embodiment, the backing layer is insoluble in water and/or an aqueous liquid such as saliva. In one embodiment, the backing layer does not dissolve in water and/or an aqueous liquid such as saliva for a period of 30 min to 75 min, preferably at least 60 min. In one embodiment, the backing layer and/or backing layer polymer mixture comprise(s) a water-insoluble polymer and/or a cellulose derivative, e.g. hydroxyethyl cellulose, preferably a water-insoluble cellulose derivative, more preferably ethyl cellulose, and thus the dissolution of the backing layer in the oral cavity is prevented. In one embodiment, the backing layer and/or backing layer polymer mixture comprise(s) a water-insoluble polymer and/or a polymer having low water solubility, preferably a water-insoluble polymer such as ethyl cellulose. In one embodiment, the backing layer polymer mixture comprises at least one component, preferably all components of a backing layer. In one embodiment, the backing layer is organic solvent based, e.g. ethanol based, and/or the mucoadhesive layer is water based.

The term "water-insoluble polymer", as used herein, relates to a polymer which is not soluble in water, e.g. ethyl cellulose and poly(methyl methacrylate). The term "polymer having low water solubility", as used herein, relates to a polymer which has negligible and/or low solubility in water, e.g. hydroxyethyl cellulose. In one embodiment, a "water-insoluble polymer" and a "polymer having low water solubility" are polymers that prevent a dissolution of the mucoadhesive film during its application in a body cavity, preferably buccal cavity, for at least 30 min. In one embodiment, the water-insoluble polymer and/or the polymer having low water solubility are selected such that a film application time of at least 15 min, preferably in the range of from about 30 min to about 75 min is provided, preferably without dissolution of the film. In one embodiment, the water-insoluble polymer is ethyl cellulose and/or poly(methyl methacrylate). In one embodiment, the cellulose derivative comprised by a backing layer is hydroxyethyl cellulose and/or ethyl cellulose. In one embodiment, the backing layer is configured to be insoluble in an aqueous liquid for at least 30 min, preferably at least 60 min. In one embodiment, the backing layer is configured to be impermeable for a macrolide for at least 30 min, preferably at least 60 min; preferably configured to be impermeable for a macrolide for at least 30 min, preferably at least 60 min in an aqueous liquid, such as saliva. In one embodiment, the backing layer and/or backing layer polymer mixture comprise(s) polymers that are insoluble in an aqueous liquid for at least 30 min. In one embodiment, the backing layer comprises ethyl cellulose, hydroxyethyl cellulose, and/or poly(methyl methacrylate), and optionally further comprises a plasticizer. In one embodiment, the backing layer comprises ethyl cellulose and/or poly(methyl methacrylate), and optionally further comprises a plasticizer.

The term "intermediate layer", as used herein, relates to a layer of a mucoadhesive film, preferably a layer arranged between a mucoadhesive layer and a backing layer. In one embodiment, the intermediate layer is an adhesive layer. In one embodiment, the intermediate layer facilitates adhesion of the mucoadhesive layer and the backing layer. In one embodiment, the intermediate layer prevents migration of the macrolide into the backing layer. In one embodiment, a film comprises an intermediate layer between the mucoadhesive layer and the backing layer to prevent migration of macrolide into the backing layer. In one embodiment, the terms "adhesive interlayer" and "intermediate layer" are used interchangeably. In one embodiment, a mucoadhesive layer and a backing layer are connected via an intermediate layer. In one embodiment, in a method of the invention, a film comprising said backing layer and said intermediate layer is combined with the mucoadhesive layer after drying the layer(s) to prevent migration of macrolide into the adhesive interlayer and/or backing layer. In one embodiment, incorporating an intermediate layer in a film of the invention allows to prevent dissolution of the macrolide in the backing layer during the method of preparing the film. In one embodiment, the intermediate layer prevents that the amount of macrolide in the mucoadhesive layer decreases and/or that the macrolide degrades during a process of preparing a backing layer on a mucoadhesive layer. In one embodiment, the intermediate layer prevents that macrolide is dissolved in ethanol comprised by a backing layer. In one embodiment, a mucoadhesive film comprising non-nanoparticulate macrolide in the form of micronized macrolide, preferably micronized crystalline macrolide, and/or in a molecularly dissolved non-nanoparticulate form, comprises an intermediate layer. In one embodiment, the intermediate layer comprises a polymer, optionally an adhesive polymer. In one embodiment, the intermediate layer comprises a polymer comprising polyvinyl acetate and/or polyvinylpyrrolidone; for example selected from poly(vinylpyrrolidone), poly[(vinylpyrrolidone)-co-(vinyl acetate)], e.g. a copolymer of 6 parts polyvinyl pyrrolidone and 4 parts polyvinyl acetate, and a mixture of poly(vinylacetate) and poly(vinyl pyrrolidone), e.g. a 8:2 mixture of poly(vinylacetate) and poly(vinyl pyrrolidone).

The term "solid solution", as used herein, relates to the macrolide being dissolved on a molecular level in a diluent or matrix in the solid state, preferably being dissolved on a molecular level in a polymer matrix. In one embodiment, the term "solid solution" relates to the macrolide being dissolved, and optionally embedded, in a polymer matrix. In one embodiment, macrolide in a molecularly dissolved non-nanoparticulate form relates to a solid solution of the macrolide. In one embodiment, the terms "solid solution" and "molecularly dissolved non-nanoparticulate form" are used interchangeably. In one embodiment, a solid solution is molecularly dissolved non-nanoparticulate macrolide provided in a polymer matrix, preferably comprising at least one polymer soluble in an organic solvent. In one embodiment, molecularly dissolved non-nanoparticulate macrolide is provided in a polymer matrix, preferably comprising at least one polymer soluble in an organic solvent. In one embodiment, a polymer soluble in organic solvents, e.g. a copolymer of methyl vinyl ether and maleic anhydride, is used to achieve an appropriate mucoadhesion of a water-free mucoadhesive film formulation, e.g. for a solid solution. In one embodiment, molecularly dissolved non-nanoparticulate macrolide is in the form of a solid solution of macrolide. In one embodiment, the polymer soluble in an organic solvent is any polymer soluble in an organic solvent selected from a) amphiphilic polymers and hydrophilic polymers, b) poly(methacrylates), c) crosslinked polyacrylic acid polymers, d) copolymers of methyl vinyl ether and maleic anhydride, e) polymers comprising polyvinyl acetate and/or polyvinylpyrrolidone, f) cellulose derivatives, and combinations thereof. In one embodiment, the polymer soluble in an organic solvent is an amphiphilic or hydrophilic polymer, e.g. Soluplus^{®}; a poly(methacrylate), e.g. a methacrylic acid copolymer; a cellulose derivative, preferably hydroxypropyl cellulose (HPC) or ethyl cellulose (EC), more preferably HPC; a crosslinked polyacrylic acid polymer, e.g. Carbopol^{®}; a copolymer of methyl vinyl ether and maleic anhydride, e.g. Gantrez^{™}; and/or a polymer comprising polyvinyl acetate and/or polyvinylpyrrolidone, e.g. Kollidon^{®}. In one embodiment, the solid solution of macrolide comprises HPC; a crosslinked polyacrylic acid polymer, e.g. Carbopol^{®}; a copolymer of methyl vinyl ether and maleic anhydride, e.g. Gantrez^{™}; and/or a polymer comprising polyvinyl acetate and/or polyvinylpyrrolidone, e.g. Kollidon^{®}. In one embodiment, the solid solution comprises HPC; and a crosslinked polyacrylic acid polymer, e.g. Carbopol^{®}, and/or a copolymer of methyl vinyl ether and maleic anhydride, e.g. Gantrez^{™}. In one embodiment, said non-nanoparticulate macrolide is present in said mucoadhesive layer in the form of micronized macrolide, preferably micronized crystalline macrolide, and/or in a molecularly dissolved non-nanoparticulate form, preferably a solid solution of molecularly dissolved macrolide. The term "molecularly dissolved non-nanoparticulate form", as used herein, relates to the macrolide being dissolved on a molecular level, e.g. dissolved on a molecular level in a polymer matrix. In one embodiment, the mucoadhesive layer comprises at least one component, preferably polymer, selected from a) amphiphilic polymers and hydrophilic polymers, b) poly(methacrylates), c) crosslinked polyacrylic acid polymers, d) copolymers of methyl vinyl ether and maleic anhydride, e) polymers comprising polyvinyl acetate and/or polyvinylpyrrolidone, f) cellulose derivatives, and combinations thereof; preferably comprises at least one component selected from a)-e), and further comprises a cellulose derivative; wherein, preferably, if said layer comprises molecularly dissolved non-nanoparticulate macrolide, said at least one component is a polymer soluble in an organic solvent, preferably ethanol, acetone, and/or isopropanol. In one embodiment, the mucoadhesive layer comprises a plasticizer. In one embodiment, the mucoadhesive layer comprises non-nanoparticulate micronized crystalline macrolide, and/or molecularly dissolved non-nanoparticulate macrolide.

In one embodiment, if said non-nanoparticulate macrolide is present in said layer in the molecularly dissolved non-nanoparticulate form, the mucoadhesive polymer and/or cellulose derivative is a film-forming polymer, preferably a film-forming polymer soluble in an organic solvent, such as ethanol. In one embodiment, if said non-nanoparticulate macrolide is present in said layer in the molecularly dissolved non-nanoparticulate form, the molecularly dissolved non-nanoparticulate form is solubilized in an organic solvent. In one embodiment, if said non-nanoparticulate macrolide is present in said layer in the molecularly dissolved non-nanoparticulate form, the cellulose derivative is selected from HPC, EC, and combinations thereof. In one embodiment, if said non-nanoparticulate macrolide is present in said layer in the molecularly dissolved non-nanoparticulate form, the mucoadhesive polymer is selected from amphiphilic or hydrophilic polymers, e.g. Soluplus^{®}; crosslinked polyacrylic acid polymers, e.g. Carbopol^{®}; copolymers of methyl vinyl ether and maleic anhydride, e.g. Gantrez^{™}; polymers comprising polyvinyl acetate and/or polyvinylpyrrolidone, preferably polymers comprising polyvinyl acetate and/or polyvinylpyrrolidone, e.g. Kollidon^{®}; and cellulose derivatives, e.g. HPC, EC, and combinations thereof.

In one embodiment, for molecularly dissolving the macrolide, the macrolide is dissolved in the organic solvent in a concentration of about 10 mg/ml to about 500 mg/ml. The term "organic solvent", as used herein, relates to any organic solvent known to a person skilled in the art. In one embodiment, the organic solvent is an ICH class 3 or ICH class 2 solvent. In one embodiment, the organic solvent is non-toxic and/or volatile. In one embodiment, an organic solvent is selected from ethanol, methanol, acetone, tetrahydrofuran, acetic acid, acetonitrile, anisole, 1-Butanol, 2-Butanol, butyl acetate, chloroform, cyclohexan, 1,1,-diethoxypropane, 1,1-dimethoxymethane, 1,2-dimethoxyethane, 1,4-dioxane, 2,2-dimethoxypropane, dichlormethan, diethyl ether, di-iso-propyl ether, dimethyl sulfoxide, dimethylformamide, 2-ethoxyethanol, ethyl acetate, ethyl formate, ethylenglycol (1,2-Ethandiol), formic acid, heptane, hexane, isobutyl acetate, isopropyl acetate, 2-methoxyethanol, 2-methyl-1-propanol, 3-methyl-1-butanol, 1-methyl-2-pyrrolidone, methyl acetate, methyl t-butyl ether, methylbuylketon, methylcyclohexane, methylethyl ketone (MEK), methylisobutyl ketone, methylisopropyl ketone, methylthetrahydrofuran, n-methylpyrrolidone, 1-pentanol, 1-propanol, 2-propanol, pentane, petroleum ether, propyl acetate, pyridine, sulfolane, t-butyl alcohol, 2,2,4-trimethylpentan (i-Octan), toluol, trichloroacetic acid, trichloroethylene, trifluroacetic acid, xylol, and combinations thereof; preferably is selected from acetic acid, acetone, anisole, 1-butanol, 2-butanol, butyl acetate, tert-butylmethyl ether, DMSO, ethanol, ethyl acetate, ethyl ether, ethyl formate, formic acid, heptane, isobutyl acetate, isopropyl acetate, methyl acetate, 3-methyl-1-butanol, methylethyl ketone, 2-methyl-1-porpanol, 2-methyltetrahydrofuran, pentane, 1-pentanol, 1-propanol, 2-propanol, propyl acetate, trimethylamine; more preferably is selected from ethanol, acetone, and isopropanol.

The term "mucoadhesive polymer", as used herein, relates to a polymer that facilitates and/or mediates mucoadhesion. In one embodiment, a mucoadhesive polymer is selected from a) amphiphilic polymers and hydrophilic polymers, b) poly(methacrylates), c) crosslinked polyacrylic acid polymers, d) copolymers of methyl vinyl ether and maleic anhydride, e) polymers comprising poly(vinyl acetate) and/or poly(vinylpyrrolidone), and mucoadhesive cellulose derivatives. In one embodiment, the mucoadhesive polymer is selected from poly(acrylic acid), e.g. Carbopol^{®} 934 or Carbopol^{®} 971 NF; crosslinked poly(acrylic acid), e.g. polycarbophil; amylopectin, e.g. Proloc^{™} 15; poly(methacrylic acid), e.g. Eudragit^{®} L100 or Eudragit^{®} S100; poly(methacrylate), e.g. Eudragit^{®} E100, Eudragit^{®} RL, or Eudragit^{®} RS; poly[(maleic anhydride)-co-(vinyl methyl ether)] or a salt thereof, e.g. Gantrez^{™} AN 119, Gantrez^{™} AN 139, Gantrez^{™} AN 149, Gantrez^{™} AN 169, or Gantrez^{™} MS-955; gelatine; polysaccharides, e.g. alginates, chitosan, xanthan gum, hyaluronic acid, pectin, or pullulan; cellulose derivatives, e.g. sodium carboxymethylcellulose (CMC), hydroxypropylmethylcellulose (HPMC), hydroxypropylcellulose (HPC), hydroxyethyl methyl cellulose (HEMC), hydroxyethylcellulose (HEC), or methylcellulose (MC); poly(vinyl pyrrolidone), e.g. Kollidon^{®} 30LP or Kollidon^{®} 90F; poly[(vinylpyrrolidone)-co-(vinyl acetate)], e.g. Kollidon^{®} VA64; a mixture of poly(vinylacetate) and poly(vinyl pyrrolidone), e.g. Kollidon^{®} SR; poly(vinyl alcohol); and poly(vinyl acetate).

The term "amphiphilic polymers", as used herein, relates to any amphiphilic polymer, preferably mucoadhesive amphiphilic polymer, known to a person skilled in the art. Typically, and amphiphilic polymer has a hydrophilic (polar) moiety and a hydrophobic (non-polar) moiety. In one embodiment, amphiphilic polymers are selected from polyvinyl caprolactam-polyvinyl acetate-polyethylene glycol graft copolymers, e.g. Soluplus^{®}, polyoxyl castor oils, and D-α-tocopherol-polyethylenglycol-succinate (TPGS).

In one embodiment, a hydrophilic polymer is a polymer that contains polar or charged groups. In one embodiment, these groups are non-ionic, anionic, cationic and/or zwitterionic. In one embodiment, the hydrophilic polymer is soluble in water. For example, the hydrophilic polymer can be selected from starch and starch derivatives, dextran, cellulose and cellulose derivatives, such as carboxymethylcellulose, hydroxypropylcellulose, hydroxyethyl cellulose, hydroxypropyl methyl cellulose, hydroxypropylethyl cellulose, sodium carboxymethyl cellulose, ethyl or propyl cellulose, polyacrylic acid, polyacrylate, polyvinylpyrrolidone, polyethylene glycol/polyvinyl alcohol copolymer, polyvinyl alcohol, polyethylene oxide polymers, polyethylene oxide/polyethylene glycol copolymers, polyacrylamide, polyethylene glycol, gelatin, collagen, alginate, pectin, pullulan, traganth, chitosan, alginic acid, arabinogalactan, galactomannan, agar-agar, agarose, carrageenan, shellac, natural gums and/or copolymers thereof.

In one embodiment, poly(methacrylates) are selected from neutral protonizable poly(methacrylates) and cationic poly(methacrylates), preferably selected from copolymers of dialkylaminoethyl methacrylates and methacrylic acid ester(s), and copolymers of trialkylammonioethyl methacrylates and methacrylic acid ester(s), in particular copolymers of dimethylaminoethyl methacrylates and methacrylic acid esters, and copolymers of trimethylammonioethyl methacrylates and methacrylic acid esters. For example, a poly(methacrylate) may be Eudragit^{®} L100, Eudragit^{®} S100, Eudragit^{®} E100, Eudragit^{®} RL, or Eudragit^{®} RS.

The term "crosslinked polyacrylic acid polymers", as used herein, relates to polyacrylic acid polymers and crosslinked polyacrylic acid polymers, preferably crosslinked polyacrylic acid polymers, known to a person skilled in the art, such as a homopolymers, copolymers, and interpolymers, e.g. acrylic acid crosslinked with allyl sucrose or allyl pentaerythritol, acrylic acid and C₁₀-C₃₀ alkyl acrylate crosslinked with allyl pentaerythritol, and carbomer homopolymer or copolymer that contains a block copolymer of polyethylene glycol and a long chain alkyl acid ester. For example, a crosslinked polyacrylic acid polymer may be Carbopol^{®} 934 or Carbopol^{®} 971 NF.

The term "copolymers of methyl vinyl ether and maleic anhydride", as used herein, relates to any copolymer of methyl vinyl ether and maleic anhydride/maleic acid known to a person skilled in the art, e.g. copolymers of monoalkyl esters of poly (methyl vinyl ether/maleic acid) with varying ester groups. In one embodiment, copolymers of methyl vinyl ether and maleic anhydride are in the form of an anhydride or in a hydrolysed form, such as a mixed sodium and calcium salt of methyl vinyl ether and maleic anhydride e.g. Gantrez^{™} MS-955 polymer. In one embodiment, the copolymers of methyl vinyl ether and maleic anhydride are selected from poly(methyl vinyl ether-co-maleic anhydride), poly(methyl vinyl ether-co-maleic acid), monoethyl ester of poly(methylvinyl ether/maleic acid), mixture of monoethyl ester of poly(methylvinyl ether/maleic acid) and monobutyl ester of poly(methylvinyl ether/maleic acid), and mixed sodium/calcium salts of poly(methylvinyl ether/maleic anhydride). In one embodiment, a copolymer of methyl vinyl ether and maleic anhydride, e.g. Gantrez^{™}, is a mixed sodium and calcium salt of a copolymer of methyl vinyl ether and (hydrolyzed) maleic anhydride. In one embodiment, the copolymer of methyl vinyl ether and maleic anhydride is a complexing agent. For example, a copolymer of methyl vinyl ether and maleic anhydride may be Gantrez^{™} AN 119, Gantrez^{™} AN 139, Gantrez^{™} AN 149, Gantrez^{™} AN 169, Gantrez^{™} AN-903, Gantrez^{™} S-96, Gantrez^{™} S-97, Gantrez^{™} ES-225, Gantrez^{™} ES-425, or Gantrez^{™} MS-955.

The terms "polymers comprising polyvinyl acetate and/or polyvinylpyrrolidone" and "polymers comprising polyvinyl acetate and polyvinylpyrrolidone", as used herein, relate to any polymers comprising polyvinyl acetate and/or polyvinylpyrrolidone known to a person skilled in the art, e.g. poly(vinylpyrrolidone), poly[(vinylpyrrolidone)-co-(vinyl acetate)], and a mixture of poly(vinylacetate) and poly(vinyl pyrrolidone). In one embodiment, the polymer comprising polyvinyl acetate and/or polyvinylpyrrolidone is selected from poly(vinylpyrrolidone), poly[(vinylpyrrolidone)-co-(vinyl acetate)], e.g. a copolymer of 6 parts polyvinyl pyrrolidone and 4 parts polyvinyl acetate, and a mixture of poly(vinylacetate) and poly(vinyl pyrrolidone), e.g. a 8:2 mixture of poly(vinylacetate) and poly(vinyl pyrrolidone). For example, polymers comprising polyvinyl acetate and/or polyvinylpyrrolidone may be Kollidon^{®} 30LP, Kollidon^{®} 90F, Kollidon^{®} VA64, or Kollidon^{®} SR.

The term "cellulose derivative", as used herein, relates to cellulose and its derivatives. In one embodiment, the cellulose derivative is preferably a cellulose derivative that swells rather than dissolves upon contact with water and/or saliva. In one embodiment, the cellulose derivative is selected from hydroxypropyl cellulose (HPC), carboxymethyl cellulose (CMC), e.g. sodium carboxymethyl cellulose (CMC), hydroxypropyl methylcellulose (HPMC), hydroxyethyl cellulose (HEC), methyl cellulose (MC), hydroxyethyl methyl cellulose (HEMC), ethyl cellulose (EC), and a combination thereof. In one embodiment, the mucoadhesive layer comprises a cellulose derivative which is a mucoadhesive cellulose derivative, e.g. selected from hydroxypropyl cellulose (HPC), carboxymethyl cellulose (CMC), hydroxypropyl methylcellulose (HPMC), hydroxyethyl cellulose (HEC), methyl cellulose (MC), and a combination thereof. In one embodiment, a cellulose derivative soluble in an organic solvent is HPC and/or EC. In one embodiment, CMC is soluble in water but not in an organic solvent.

In a method of preparing a mucoadhesive film, the layers of the film can either be prepared individually and then joined, e.g. via an intermediate layer and/or via an adhesive agent, and/or can be prepared on top of each other, e.g., by first preparing the mucoadhesive layer and then subsequently coating the mucoadhesive layer with the backing layer, optionally by coating the mucoadhesive layer with the intermediate layer and the backing layer. For example, the method of preparing the mucoadhesive film can comprise preparing the mucoadhesive layer, the backing layer, and optionally the intermediate layer individually, and subsequently joining the layers using lamination, e.g. thermo-lamination, or compression. For example, after casting the mucoadhesive layer and subsequent drying of the layer, the second layer, e.g. intermediate layer or backing layer, is casted on top of the mucoadhesive layer. In one embodiment, the first and second layers, e.g. mucoadhesive layer and intermediate layer or backing layer, are mounted onto each other. In one embodiment, the mucoadhesive layer is not completely dried before applying the intermediate layer and/or backing layer. In one embodiment, a roll can be used to compress the layers onto each other. In one embodiment, a method of preparing a mucoadhesive film comprises preparing a mucoadhesive layer and the backing layer, and subsequently joining the mucoadhesive layer and backing layer, e.g., by compressing or adhesion. In one embodiment, a method of preparing a mucoadhesive film comprises preparing a mucoadhesive layer, an intermediate layer, and a backing layer, and subsequently joining the layers, e.g., by compressing or adhesion.

In one embodiment, said step of preparing a mucoadhesive layer and said step of preparing a backing layer are followed by a step of joining said mucoadhesive layer and said backing layer by compression and/or lamination, e.g. thermo-lamination. Optionally, said mucoadhesive layer and/or said backing layer are dried or partially dried prior to said step of joining said mucoadhesive layer and said backing layer. In one embodiment, said step of preparing a mucoadhesive layer, said step of preparing an intermediate layer, and said step of preparing a backing layer are followed by a step of joining said mucoadhesive layer, said intermediate layer, and said backing layer by compression and/or lamination, e.g. thermo-lamination. Optionally, said mucoadhesive layer, said intermediate layer, and/or said backing layer are dried or partially dried prior to said step of joining said mucoadhesive layer, said intermediate layer, and said backing layer. In one embodiment, said joining is performed by compression and/or lamination, e.g. thermo-lamination.

In one embodiment, said backing layer is prepared on a surface of said mucoadhesive layer, e.g. by coating said polymer mixture on a surface of said mucoadhesive layer, or is prepared separately and is subsequently joined with said mucoadhesive layer, optionally joined via an intermediate layer. In one embodiment, if said mucoadhesive layer and said backing layer, optionally said intermediate layer, are prepared separately, said layers are prepared on a process liner. In one embodiment, if said mucoadhesive layer comprises micronized macrolide, preferably micronized crystalline macrolide, said backing layer is prepared on a surface of said mucoadhesive layer, e.g. by coating said polymer mixture on a surface of said mucoadhesive layer. In one embodiment, if said mucoadhesive layer comprises molecularly dissolved macrolide, said backing layer is prepared separately from said mucoadhesive layer, and the mucoadhesive layer and backing layer are subsequently joined after their preparation, optionally joined via an intermediate layer. In one embodiment, the mucoadhesive layer, the backing layer, and optionally the intermediate layer, are each prepared on a process liner, and are subsequently joined after their preparation.

In one embodiment, the method of preparing a mucoadhesive film comprises a step of joining the mucoadhesive layer, the backing layer, and optionally the intermediate layer, e.g. by compression and/or lamination, e.g. thermo-lamination. In one embodiment, said mucoadhesive layer, backing layer, and optionally intermediate layer, are dried or partially dried prior to a step of joining the layers. In one embodiment, the mucoadhesive layer, backing layer, and optionally intermediate layer, are prepared separately and are subsequently joined after their preparation, and/or are prepared on top of each other.

In one embodiment, the mucoadhesive layer and/or film is/are for use in medicine. In one embodiment, said use involves an attachment of said film in a body cavity, preferably the buccal cavity, of a patient, wherein said attachment is such that said mucoadhesive film makes contact with a site of attachment in the body cavity, preferably buccal cavity, via a side of said polymeric matrix layer that has no backing layer attached.

The mucoadhesive film is advantageous in that the rich blood circulation of the oral and/or buccal mucosa ensures a rapid transfer of the active substance into the blood circulation. Furthermore, the film and/or layer of the invention is advantageous in that the active ingredient is largely absorbed through the mucous membrane and thus the "first-pass metabolism", which occurs in the conventional delivery form of an active ingredient in tablet form, is avoided. Further, such the mucoadhesive film of the invention has the advantage that the macrolide is protected from degradation due to pH and digestive enzymes of the gastrointestinal tract. The mucoadhesive layer and/or film further provide(s) a rapid onset of action, e.g. relative to the oral route. A mucoadhesive film is an easy way of drug administration and therefore especially suitable for use in pediatrics. Furthermore, the mucoadhesive film avoids hurdles related to drug administration via the nasogastric tube and is flexible in physical shape, state, size, and surface. The mucoadhesive film is further advantageous in that it allows an accurate dosing.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

### BRIEF DESCRIPTION OF THE FIGURES

The present invention is now further described by reference to the following figures.

All methods mentioned in the figure descriptions below were carried out as described in detail in the examples.
**Figure 1** shows a schematic representation of the method of producing a mucoadhesive layer and/or mucoadhesive film of the invention.
**Figure 2** shows the release and unidirectional release of the film comprising micronized crystalline macrolide (L135) and a film having the same composition but without Soluplus^{®}.
**Figure 3** shows an overlay of XRPD spectra of and L135 (3), TiO₂ (2) and pure crystalline Tacrolimus (1).
**Figure 4** shows the in vivo macrolide blood level after administration of the mucoadhesive film.
**Figure 5** shows a schematic representation of the method of producing a mucoadhesive film of the invention which comprises a mucoadhesive layer, an intermediate layer (adhesive layer), and a backing layer.

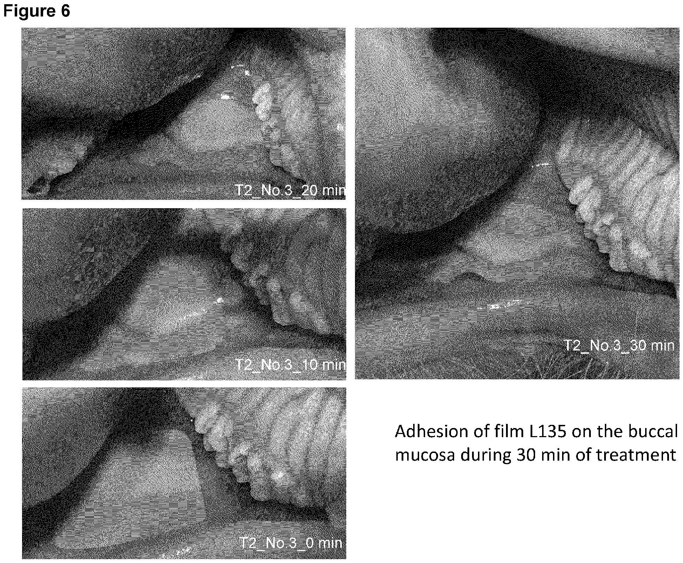

**Figure 6** shows pictures of a film comprising micronized crystalline macrolide (L135) adhered onto the buccal mucosa during 30 min of treatment. Successful adhesion and treatment were also observed with the film comprising molecularly dissolved non-nanoparticulate macrolide (171Tac0051).

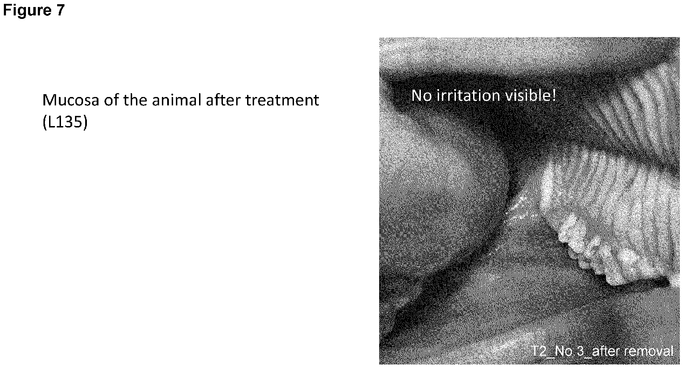

**Figure 7** shows the mucosa of the animal from figure 6 after treatment and after removal of the film L135. No irritation of the mucosa is visible.

In the following, reference is made to the examples, which are given to illustrate, not to limit the present invention.

### EXAMPLES

### Example 1: Preparation of a mucoadhesive film with micronized crystalline macrolide

A polymer mixture was prepared by mixing water with acetone, and by dispersing titanium dioxide in the mixture while stirring. Glycerol and Soluplus^{®} were added. Hydroxypropyl cellulose (HPC) powder was then slowly added, and sodium carboxymethyl cellulose (CMC) powder was added after complete dissolution of HPC. The viscosity of the mixture increased with dissolution of CMC. To avoid air bubbles in the polymer mixture, the stirring speed was reduced.

The micronized macrolide was predispersed in 50 mM sodium acetate buffer with pH 4, and then added to the polymer mixture. The crystals of the micronized macrolide had D₁₀ 2 µm; D₅₀ 10 µm; D₉₀ 25 µm.

300 g of the mixture were prepared. The concentration of solids in the mixture was 15% by weight.

**Table 1: Weighed portions for the production of the mucoadhesive layer (L134).**

| **L134** | **Weighed portion [g]** | **Solid [wt.-%]** |
|---|---|---|
| Tacrolimus | 3.68644 | 8.0% |
| Buffer | 106.8 | |
| TiO₂ | 1.14903 | 2.5% |
| Glycerol | 5.53318 | 12.1% |
| Soluplus^{®} | 11.12 | 24.2% |
| HPC | 14.59 | 31.8% |
| CMC | 9.81 | 21.4% |
| Acetone | 10.75 | |
| Water | 136.7 | |
| **Total** | **300** | **100%** |
| Solid content | 15% | |

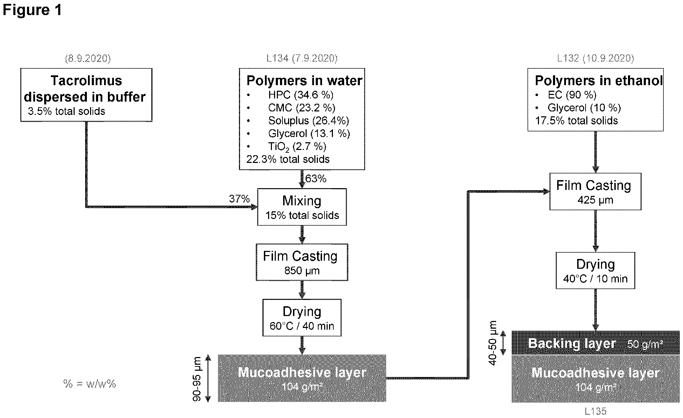

The obtained mixture was coated onto a process liner FL2000 in a thickness of 850 µm (thickness in undried form) at a speed of 5 mm/s with a double skrew coating knife and then dried at 60°C for 40 minutes (Figure 1). Several sheets were produced. The targeted area weight of the film was about 104 g/m². Die-cuts of 6 cm² size were prepared, and the area weight of the die-cuts was determined:

**Table 2: Weight per unit area of the mucoadhesive layer.**

| **Sheet** | **Weight per unit area mucoadhesive layer** |
|---|---|
| L134-1 | 106.7 g/m² (RSD 3.32 %) |
| L134-2 | 108.3 g/m² (RSD 2.74 %) |
| L134-3 | 110.2 g/m² (RSD 3.49 %) |

| | |
|---|---|
| RSD: relative standard deviation of 10 samples | |

### Example 2: Sealing of the mucoadhesive layer with a backing layer L132

The polymer mixture for sealing was prepared. Ethanol was mixed with glycerol (as a plasticizer) under agitation. Ethyl cellulose powder was then slowly added and the mixture was stirred until homogenization.

300 g of the mixture were prepared. The concentration of solids in the mixture was 17.5 % by weight.

**Table 3: Weighed portions for the production of the backing layer (L132).**

| **L132** | **Weighed portion [g]** | **Solid [wt.-%]** |
|---|---|---|
| EC | 47.69 | 90.1% |
| Glycerol | 5.2431 | 9.9% |
| Ethanol | 247.09 | |
| **Total** | **300** | **100%** |
| Solid content | 17.6% | |

After drying of the mucoadhesive layer, the mucoadhesive layer sheets were coated with a solution of ethyl cellulose in ethanol in a thickness of 425 µm at a speed of 5 mm/s using a double skrew coating knife and then dried for 10 minutes at 40°C (Figure 1). The targeted area weight of the film was about 50 g/m². Die-cuts of 6 cm² size were prepared, and the area weight of the die-cuts was determined:

**Table 4: Weight per unit area of the mucoadhesive film.**

| **Sheet** | **Sheet** | **Area weight mucoadhesive layer L134** | **Area weight backing layer L132** | **Area weight film L135** |
|---|---|---|---|---|
| L135-4 | L134-1 | 106.7 g/m² (RSD 3,32 %) | 43.9 g/m² | 150.6 g/m² (RSD 1.22%) |
| L135-5 | | | 43.6 g/m² | 150.3 g/m² (RSD 2.09 %) |
| L135-2 | L134-2 | 108.3 g/m² (RSD 2,74 %) | 47.4 g/m² | 155.7 g/m² (RSD 1.30 %) |
| L135-3 | L134-3 | 110.2 g/m² (RSD 3,49 %) | 44.0 g/m² | 154.2 g/m² (RSD 1.34 %) |

| | | | | |
|---|---|---|---|---|
| RSD: relative standard deviation of 10 samples | | | | |

Square films with rounded corners of 6 cm² area and 2.48 cm edge length were punched out of the two-layer laminate comprising the mucoadhesive layer and the backing layer. The process liner was removed and the film was packed in air- and moisture protected individual sachets of size 8 cm x 9.6 cm. Approximately 70 laminates were produced.

### Example 3: Preparation of a mucoadhesive film with solid solution of macrolide

### Solubility of tacrolimus

Tacrolimus was dissolved in an organic solvent. Particularly, the saturation solubility of tacrolimus in different organic solvents was tested. Crystalline active ingredient was added to a defined amount of solvent at room temperature while stirring until an insoluble sediment was formed. After filtration, the concentration of active ingredient was quantified by HPLC in the supernatant.

Tacrolimus was soluble in all tested organic solvents. For the further experiments relating to a solvent-based mucoadhesive film, ethanol was chosen as organic solvent. Non-toxicity and low boiling point - and thus the possibility of complete removal by drying at low temperatures - characterize the solvent ethanol.

**Table 5: saturation solubility of tacrolimus.**

| | **Solubility / mg/ml** | **Boiling point / °C** |
|---|---|---|
| Water | 0.00076 | 100 |
| Acetic Acid | 545 | 118 |
| Ethanol | 389 | 78 |
| iso-Propanol | 243 | 82,5 |
| DMSO | 582 | 189 |

### Polymer matrix for solid solution

For developing a mucoadhesive film comprising macrolide as solid solution, polymers were chosen that are soluble in organic solvents. Thus, it was possible to work in a water-free system. Sample films with an area of 6 cm² were prepared from each of the matrices and tested for water solubility and adhesion.

For example, polymers soluble in ethanol having film-forming and mucoadhesive characteristics are crosslinked polyacrylic acid polymers, e.g. Carbopol^{®}; copolymers of methyl vinyl ether and maleic anhydride, e.g. Gantrez^{™}; poly(methacrylates), e.g. Eudragit^{®}; polymers comprising polyvinyl acetate and/or polyvinylpyrrolidone, e.g. Kollidon^{®} SR (8:2 mixture of poly(vinylacetate) and poly(vinyl pyrrolidone)).

Various combinations of polymers were tested:

**Table 6: Development of an ethanol-based mucoadhesive layer.**

| **Ingredient/Excip ient** | **171Tac0008** | **171Tac0016** | **171Tac00xx** | **171Tac0020** | **171Tac0023** | **171Tac0045** | **171Tac0051** |
|---|---|---|---|---|---|---|---|
| HPC Klucel EF | 54.9% | 56.2% | 56.2% | 51.7% | 42.7% | 42.7% | 42.7% |
| HPC Klucel GF | 7.0% | 7.0% | 7.0% | 10.0% | 10.0% | 10.0% | 10.0% |
| Carbopol 934 | 15.0% | | 7.5% | | | | |
| Gantrez AN 169 | | 15.0% | 7.5% | 8.5% | 8.5% | | |
| Gantrez MS-955 | | | | | | 4.0% | 4.0% |
| EC (Aqualon N₅₀NF) | | | | 10.0% | 30.0% | 34.5% | 34.5% |
| Glycerol | 18.0% | 18.0% | 18.0% | 16.0% | 5.0% | 5.0% | 5.0% |
| Titandioxid | 3.8% | 3.8% | 3.8% | 3.8% | 3.8% | 3.8% | 3.8% |
| NaCl | 1.3% | | | | | | |
| **SUM** | **100%** | **100%** | **100%** | **100%** | **100%** | **100%** | **100%** |
| Solid content | | | | | | | 20.0% |
| Area weight /g/m² | 104 | | | | | | 150 |

The tested combinations successfully showed mucosa adhesion. Furthermore, the tested polymer combinations provided low water solubility.

### Preparation of the mucoadhesive layer 171Tac0051

An ethanol based polymer mixture was prepared. Titanium dioxide was dispersed in the ethanol under agitation by stirring. Glycerol was added. The powdered polymers were slowly added, and stirring continued until complete dissolution. The active ingredient was dissolved in ethanol and stirred into the prepared polymer mixture to obtain a layer composition.

The layer composition was coated on a process liner (FO Scotchpak 9755) and subsequently dried. The targeted are weight of the film was about 150 g/m². 500 g of the mixture were prepared. The concentration of solids in the mixture was 20 % by weight.

### Preparation of the backing layer

The polymer mixture for sealing was prepared as described above. 1000 g of the mixture were prepared. The concentration of solids in the mixture was 15 % by weight.

The mixture was coated on a process liner and subsequently dried. The targeted are weight of the film was about 50 g/m².

**Table 7: Weighed portions for the production of the backing layer 171Tac0003.**

| **171Tac0003** | **Weighed portion [g]** | **Solid [wt.-%]** |
|---|---|---|
| EC | 135 | 90% |
| Glycerol | 15 | 10% |
| Ethanol | 850 | |
| **Total** | **1000** | **100%** |
| Solid content | 15% | |

The backing layer and the mucoadhesive layer were joined via an adhesive intermediate layer.

### Example 4: Preparation of an intermediate layer 171Tac0066

A mixture was prepared, coated on a process liner (FO Scotchpak 9755), and then dried. The targeted are weight of the film was about 60 g/m².1000 g of the mixture were prepared. The concentration of solids in the mixture was 55 % by weight.

**Table 8: Weighed portions for the production of the intermediate layer 171Tac0066.**

| **171Tac0066** | **Weighed portion [g]** | **Solid [wt.-%]** |
|---|---|---|
| Kollidon VA64 | 440 | 80% |
| Glycerol | 110 | 20% |
| Ethanol | 550 | |
| **Total** | **1000** | **100%** |
| Solid content | 55% | |

Alternatively, the intermediate layer comprised a blend of polyvinyl acetate and povidone (K 30) in the ratio 8:2, namely Kollidon^{®} SR, instead of the vinylpyrrolidone-vinyl acetate copolymer Kollidon^{®} VA64.

The 3 layers, namely mucoadhesive layer, intermediate layer, and backing layer, were laminated on top of each other by pressure. Rectangular films with 2x3 cm edge length were punched out of the finished three-layer laminate, the film was packed in individual sachets (Hutamaki COC) to protect it from air and moisture. About 150 laminates were produced.

### Example 5: Composition of the layers

The composition of the layers is shown below:

**Table 9: Characteristics and composition of the layers**

| **Batch no** | **L135** | **171Tac0051** | **Function** |
|---|---|---|---|
| Appearance | Square-shaped with rounded corners | Rectangular | |
| Size Length x width | 6cm² 2.48 cm x 2.48 cm | 6 cm² 3 cm x 2 cm | |
| Target Area weight | 154 g/m² | 260 g/m³ | |
| Target Weight | 92.4 mg/film | 156 mg/film | |
| Target macrolide content | 5 mg/film | 5 mg/film | |

| **Mucoadhesive layer** | [mg/film] | [mg/film] | |
|---|---|---|---|
| Tacrolimus | 5.0 | 5.0 | Active Substance |
| Soluplus^{®} | 15.0 | -- | Solubilizer |
| Ethyl cellulose (Aqualon N₅₀NF) | -- | 29.3 | Matrix polymer |
| Hydropropyl cellulose EF (Klucel EF) | 19.9 | 36.3 | Matrix polymer |
| Hydropropyl cellulose GF (Klucel GF) | -- | 8.5 | Matrix polymer |
| Gantrez MS-955 | -- | 3.4 | Mucoadhesive |
| Sodium Carboxymethyl cellulose (Blanose 7LP) | 13.3 | -- | Matrix polymer |
| Glycerol | 7.7 | 4.3 | Softening agent |
| Titanium dioxide | 1.6 | 3.2 | Colorant |
| Ethanol | -- | / | Process solvent |
| Water | / | -- | Process solvent |
| **Target Area weight (dry)** | **104 g/m²** | **150 g/m²** | |

| **Adhesive interlayer** | | | |
|---|---|---|---|
| Kollidon VA64 | -- | 2.88 | Matrix polymer |
| Glycerol | -- | 0.72 | Softening agent |
| Ethanol | -- | / | Process solvent |
| **Area weight (dry)** | **--** | **60 g/m²** | |

| **Backing layer** | | | |
|---|---|---|---|
| Ethyl cellulose (Aqualon N₅₀NF) | 2.7 | 2.697 | Matrix polymer |
| Glycerol | 0.3 | 0.3 | Softening agent |
| Brilliant Blue | q.s. | 0.003 | Colorant |
| Ethanol | / | / | Process solvent |
| **Target Area weight (dry)** | **50 g/m²** | **50 g/m²** | |

### Example 6: Characterisation of the films

The prepared films were analyzed for content, release, unidirectional release, water content and solvent content. The analytical methods used are given in Table 10, the results of the surveys are given in Table 11.

To determine the macrolide release, the film was placed with the backing layer on a rotating cylinder (with the mucoadhesive layer facing the medium) and the amount of drug released into the medium at the indicated time points was quantified by HPLC. The medium used was 500 ml of phosphate buffer having pH 7 with 0.1% sodium dodecyl sulfate. The values are given as mean values; the minimum and maximum values of the n individual determinations are given in parentheses.

To determine the unidirectional release, the film was placed with the mucoadhesive layer on a rotating cylinder (with the backing layer facing the medium) and the amount of drug released into the medium was quantified after 10 min, 20 min and 30 min as described above. The medium was the same as described above.

**Table 10: Analytical methods used to characterize the films.**

| **Test** | **Analytical method** |
|---|---|
| API content | HPLC method gradient according to USP41: C18 RP HPLC column |
| | UV detection |
| | 30 or 60 min run time |
| Dissolution (Ph.Eur. 2.9.3) | Apparatus: Rotating cylinder or Paddle over sinker |
| | Strirring speed: 50 rpm, |
| | Medium: sodium hydrogen phosphate buffer pH 7, 0.1% SDS |
| | Volume: 500 ml medium |
| | HPLC method: isocratic, C18 RP HPLC column, UV detection |
| Inverse dissolution | MBF attached to the cylinder with the backing layer facing the dissolution medium |
| | Method see above |
| Water content | Karl Fischer oven method Ph.Eur. |
| Residual solvents | GC-FID method |

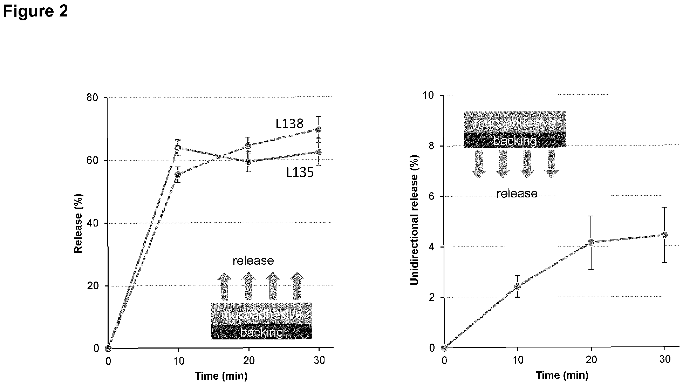

Both films, i.e. the film comprising micronized crystalline macrolide and the film comprising molecularly dissolved macrolide (L135 and 171Tac0051, respectively), successfully released the macrolide almost completely. The macrolide release of L135 (Figure 2), was not caused by the presence of Soluplus^{®} (3-fold excess to the active ingredient).

Only a small amount of tacrolimus (max. 5 %) was released via the edges of the film. The backing layer efficiently prevented the release of tacrolimus. Accordingly, a release of the active ingredient into the oral cavity - and thus a potential swallowing of the active ingredient - can be efficiently prevented by the backing layer.

A small amount of ethanol was detected in both films. The value determined was below the permissible amount defined by the ICH for ethanol and is therefore suitable for in vivo application. Acetone was not detected in either of the films.

The water content of the film comprising micronized crystalline macrolide, e.g. L135, was less than 3%; the film comprising molecularly dissolved macrolide, e.g. 171Tac0051, was produced without the use of water and thus the water content was not analyzed.

Low water content and low residual solvent content of both films prove efficient drying of the laminate.

**Table 11: Results of the characterization of the films.**

| **Batch no** | **L135** | **171Tac0051** |
|---|---|---|
| **Area weight** [g/m²] | 150.3 - 155.7 | |
| **API content** [mg/film, %] | 4.35 mg/film | 4.7 mg/film |
| | 84.4% | 94% |
| | (RSD 1.18%; n=3) | |
| **Dissolution** [%] | n=6 (Min - Max) | |
| 10 min | 64% (60-67%) | 14% (11-20%) |
| 20 min | 59% (54-64%) | 44% (39-54%) |
| 30 min | 62% (57-69%) | 57% (51-64%) |
| 60 min | -- | 69% (67-72%) |
| 120 min | -- | 69% (66-73%) |
| **Inverse dissolution** [%] | n=6 (Min - Max) | |
| 10 min | 2.4% (1.8-2.9%) | not tested |
| 20 min | 4.2% (3.1-6.1%) | not tested |
| 30 min | 4.4% (3.6-6.4%) | not tested |
| **Water content** [%] | 2.9% | not tested |
| **Residual solvents** [%] | | |
| Ethanol | 1.9% | 5.2% |
| Acetone | -- | -- |

| | | |
|---|---|---|
| RSD: relative standard deviation of n die-cuts | | |

### Example 7: Stability of the laminates

Both laminates, i.e. the film comprising micronized crystalline macrolide and the film comprising molecularly dissolved macrolide (L135 and 171Tac0051, respectively), were stored under temperature and humidity control and analytically characterized after the indicated time points. The results are reported in Table 12 and Table 13.

**Table 12: Stability of the laminate L135 when stored at room temperature.**

| **Batch no** | **L135 After production** | **L135 6 weeks at 21°C** |
|---|---|---|
| **Weight** | 91.4 mg/film (RSD 0.23%, n=3) | 90.5 mg/film (RSD 0.32%, n=3) |
| **API content** | 4.35 mg/film | 4.16 mg/film (95.8% of initial value) |
| **Dissolution** | n=6 (Min - Max) | |
| 10 min | 64% (60-67%) | 44% (29-57%) |
| 20 min | 59% (54-64%) | 57% (51-60%) |
| 30 min | 62% (57-69%) | 55% (52-58%) |

The macrolide continent of the laminate L135 decreased by only 4.2% within 6 weeks when stored at room temperature due to degradation of the active ingredient.

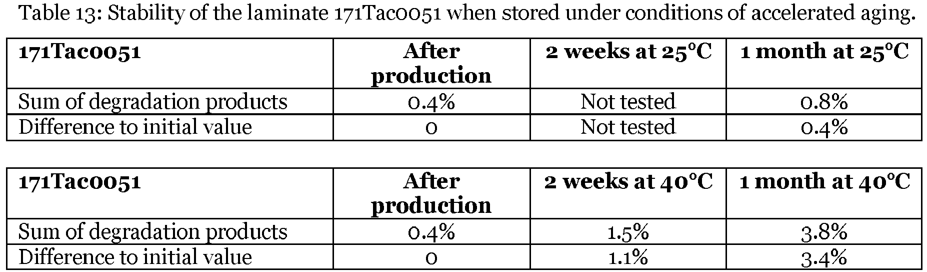

After two weeks of storage of the film comprising molecularly dissolved macrolide (laminate 171Tac0051) at 40°C, 1.5% of degradation products were detected. After production, this batch contained 0.4% degradation products. Thus, 1.1% of degradation products were formed by 2 weeks of storage at 40°C, and 3.4% of degradation products after 4 weeks of storage at 40°C.

**Table 14: Comparison of the stability of the laminates L135 and 171Tac0051.**

| **Batch no** | **Macrolide degradation** |
|---|---|
| L135 | -4.2 % in 6 weeks at room temperature |
| 171Tac0051 | -0.4 % in 4 weeks at room temperature |
| | -3.4 % in 4 weeks at 40°C |

Accordingly, the water free formulation of the film comprising molecularly dissolved macrolide (laminate 171Tac0051) even further increases the chemical stability of the film. Copolymers of methyl vinyl ether and maleic anhydride, e.g. Gantrez^{™}, even further enhances the chemical stability of the film due to its complexing and stabilizing characteristics.

### Example 8: Morphology of the active agent

The film L135 was characterized radiographically. Samples of the active ingredient, micronized Tacrolimus, and samples of the colorant titanium dioxide served as controls.

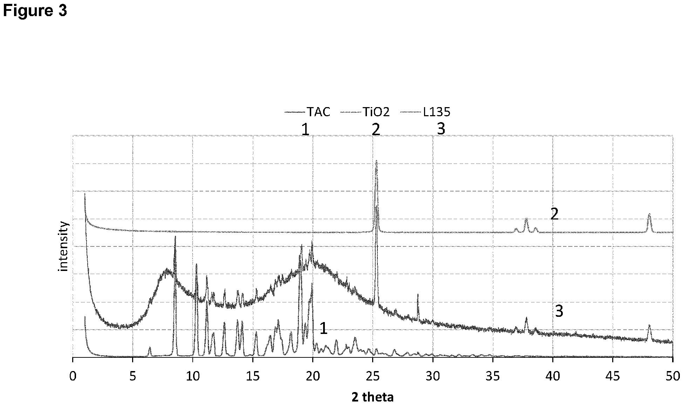

The XRPD image (Figure 3) of the film comprising micronized crystalline tacrolimus (L135; red, 3) shows the characteristic reflections of the crystalline tacrolimus (blue, 1) and the excipient TiO₂ (orange, 2). The reflection detected at about 28° can be assigned to silicon, which was used as substrate for the XRPD measurement.

As shown in Figure 3, the morphology of the micronized crystalline macrolide did not change by the incorporation of the micronized crystalline macrolide into the film (L135).

### Example 9: In vivo application (first study)

The pig was chosen as animal model, since this animal model is very similar to the functional-anatomical relationships of humans. All animals were 6 to 8 months old at the time of treatment with a body weight between 14 and 16 kg. Animals were fasting (overnight) for at least 16 hours before treatment and anesthetized during treatment. The film was placed with the mucoadhesive layer side on the mucosa in the animal's mouth and lightly pressed until the film adhered. The film remained on the buccal mucosa for 30 min and was then removed.

Blood samples were collected via a venous catheter. For determination of blood levels of tacrolimus, 2 ml of blood was collected from all animals into K2EDTA-coated tubes. Samples were collected at the following time points: immediately before administration (0 min), then 15, 30, 60, 90, 120, 180, and 240 min, and 6, 8, 12, 18, and 24 h after administration of the film. After blood collection, the blood tubes were cooled on ice and stored in a freezer (at a temperature in the range of -30°C to -15°C) until analysis. The concentration of tacrolimus was determined by a reliable LC-MS/MS method.

After application in animals, the tacrolimus content of the applied film was determined (drug content post treatment). The applied dose corresponds to the tacrolimus content of the film minus the residual content after application. The biologically active dose was calculated in relation to the weight of the individual animal (applied dose/body weight).

Pharmacokinetic parameters were calculated from the time course of blood concentration. The calculated AUC was normalized with the applied dose and set in relation to the individual weight of the animal (AUC_all / dose).

**Table 15: Application of L135 in three pigs: blood level of tacrolimus and pharmacokinetic parameters.**

| **Animal ID** | **#1** | **#2** | **#1** | **#2** | **#3** |
|---|---|---|---|---|---|
| Species | Goettingen Minipig. naïve | | | | |
| Sex | Female | | | | |
| Body weight / kg | 15.0 | 14.7 | 15.6 | 15.6 | 16.6 |
| Age / months | 7-8 | 7-8 | 8-9 | 8-9 | 8-9 |
| Date of treatment | 28.10.2020 | 28.10.2020 | 17.11.2020 | 17.11.2020 | 17.11.2020 |

| **Dosage form** | **L135** | **L135** | **Prograf capsule** | **Prograf capsule** | **Prograf capsule** |
|---|---|---|---|---|---|
| Dose / mg | 4.35 | 4.35 | 5 | 5 | 5 |
| Drug content post treatment / mg (% of dose) | 2.36 (54.3%) | 2.54 (58.4%) | -- | -- | -- |
| Applied dose / mg | 1.99 | 1.81 | 5 | 5 | 5 |

| **time / hours** | **Blood concentration / ng/ml** | | | | |
|---|---|---|---|---|---|
| 0 | 0 | 0 | 0 | 0 | 0 |
| 0.25 | 0 | 0 | 0.162 | 0.0775 | 0.177 |
| 0.5 | 0 | 0 | 0.675 | 0.96 | 0.903 |
| 1 | 0.36 | 0 | 3.39 | 3.57 | 1.78 |
| 1.5 | 0.556 | 0.0625 | 7.49 | 4.20 | 1.90 |
| 2 | 0.473 | 0.101 | 9.04 | 4.15 | 2.36 |
| 3 | 0.253 | 0.0855 | 8.40 | 3.85 | 3.20 |
| 4 | 0.158 | 0.069 | 8.54 | 2.75 | 4.03 |
| 6 | 0.209 | 0.0608 | 7.74 | 2.90 | 3.27 |
| 8 | 0.142 | 0 | 5.41 | 2.97 | 2.79 |
| 12 | 0.161 | 0 | 3.80 | 2.25 | 2.03 |
| 18 | 0.153 | 0 | 2.50 | 1.40 | 1.6 |
| 24 | 0.104 | 0 | 1.99 | 1.12 | 2.08 |

| **Pharmacokinetic parameters** | | | | | |
|---|---|---|---|---|---|
| T_max / hours | 1.5 | 2 | 2 | 1.5 | 4 |
| C_max / ng/ml | 0.556 | 0.101 | 9.04 | 4.20 | 4 |
| AUC_all / h *ng/ml | 4.18 | 0.418 | 105 | 53.1 | 54.1 |
| Applied dose/body weight / mg/kg | 0.133 | 0.123 | 0.321 | 0.321 | 0.301 |
| **AUC_all / dose (h *ng/ml / mg/kg)** | **31.5** | **3.39** | **329** | **166** | **180** |
| **Intraindividual comparison: AUC_all/dose of L135/Prograf** | **9.6%** | **2.0%** | **100%** | **100%** | |

The residual macrolide levels of the films were between 54.3% and 58.4%. Tacrolimus was successfully detected in the blood of both pigs after buccal administration; the maximum concentration was reached after 1.5 and 2 hours, respectively (t_max). The film did not cause local irritation of the buccal mucosa.

These results demonstrate that a buccal resorption of micronized crystalline macrolides is effective.

There was no treatment-related mortality, clinical signs or changes in body weights. There was no irritation at the application sites of MBF loaded with micronized tacrolimus.

Mean blood concentrations-time profiles from the three animals following buccal application of MBF loaded with micronized tacrolimus, and oral administration of a 5 mg Prograf capsule. It was successfully demonstrated that micronized tacrolimus can pass the buccal mucosa following application as MBF.

### Example 10: In vivo application (second study)

The first study was repeated with a film comprising molecularly dissolved macrolide (171Tac0051) with five further animals. The animals were not the same animals as tested in the first study. The film remained on the buccal mucosa for 60 min and was then removed. The further procedure was analogous to the first study.

**Table 16: Application of 171Tac0051 in five pigs: blood level of tacrolimus and pharmacokinetic parameters.**

| **Animal ID** | **#1** | **#2** | **#3** | **#4** | **#5** |
|---|---|---|---|---|---|
| Species | Goettingen Minipig. naïve | | | | |
| Sex | Female | | | | |
| Body weight / kg | 16.0 | 14.1 | 15.8 | 14.4 | 14.8 |
| Age / months | 6-7 | 6-7 | 6-7 | 6-7 | 6-7 |
| Date of treatment | 17.05.2021 | 17.05.2021 | 17.05.2021 | 17.05.2021 | 17.05.2021 |
| **Dosage form** | 171Tac0051 | | | | |
| Dose / mg | 4.7 | 4.7 | 4.7 | 4.7 | 4.7 |
| Drug content post treatment / mg | 2.1 | 2.2 | 1.9 | 2.3 | 2.1 |
| Applied dose / mg | 2.6 | 2.5 | 2.8 | 2.4 | 2.6 |

| **time / hours** | **Blood concentration / ng/ml** | | | | |
|---|---|---|---|---|---|
| 0.25 | 0 | 0 | 0 | 0 | 0 |
| 0.5 | 0.0916 | 0.127 | 0.201 | 0 | 0.104 |
| 1 | 0.88 | 1.08 | 1.4 | 0.655 | 1.01 |
| 1.5 | 2.3 | 2.95 | 4.24 | 1.71 | 2.52 |
| 2 | 2.74 | 4.44 | 4.72 | 2.37 | 3.89 |
| 3 | 1.9 | 4.4 | 3.52 | 1.71 | 3.98 |
| 4 | 1.2 | 2.83 | 2.02 | 0.995 | 2.87 |
| 6 | 0.655 | 1.8 | 1.08 | 0.554 | 2.67 |
| 8 | 0.406 | 0.822 | 0.756 | 0.366 | 0.948 |
| 12 | 0.29 | 0.473 | 0.468 | 0.23 | 0.643 |
| 18 | 0.188 | 0.317 | 0.313 | 0.176 | 0.417 |
| 24 | 0.132 | 0.255 | 0.245 | 0.115 | 0.306 |

| **Pharmacokinetic parameters** | | | | | |
|---|---|---|---|---|---|
| T_max / hours | 2 | 2 | 2 | 2 | 3 |
| C_max / ng/ml | 2.74 | 4.44 | 4.72 | 2.37 | 3.98 |
| AUC_all / h *ng/ml | 12.9 | 25.1 | 22.4 | 11.0 | 27.8 |
| Applied dose/body weight / mg/kg | 0.163 | 0.177 | 0.177 | 0.167 | 0.176 |
| **AUC_all / dose (h^{∗}ng/ml / mg/kg)** | **79.3** | **142** | **126** | **65.7** | **158** |

The analysis of the drug content of the films post treatment show that about 55% of the macrolide is biologically active and 45% remain in the film.

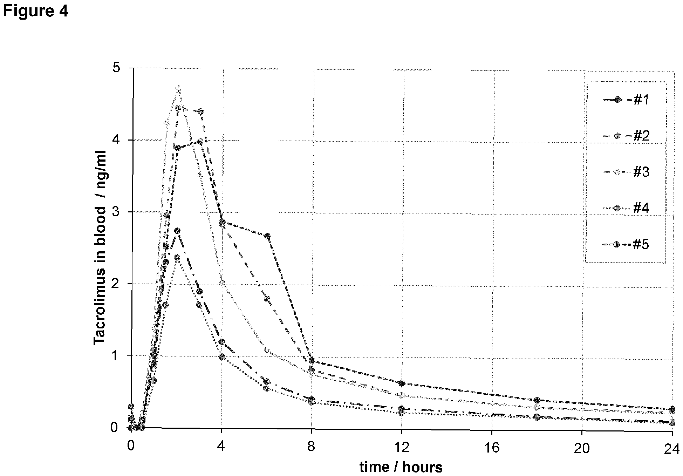

Tacrolimus was detected in the blood of all five pigs after buccal application (Figure 4). The maximum concentration was reached after 2 to 3 hours (t_max). The film did not cause local irritation of the buccal mucosa.

The blood concentrations achieved were in the range of those obtained with oral administration of the commercial product Prograf. The time necessary for reaching maximum blood levels, namely 2-3 hours, is comparable to the commercial product. Accordingly, both the film comprising micronized crystalline macrolide and the film comprising molecularly dissolved macrolide allowed to effectively administer macrolide. Furthermore, unexpectedly, the films allowed to achieve blood level concentrations of macrolide which are suitable for in vivo application to a patient.

### Example 11: Further optimization of the MBF for preventing migration of macrolide into the backing layer

The backing layer was based on ethyl cellulose dissolved in ethanol and was casted on the dried mucoadhesive layer containing Tacrolimus. Because of the excellent solubility of Tacrolimus in ethanol, a part of the active substance was extracted into the backing layer during the manufacturing.

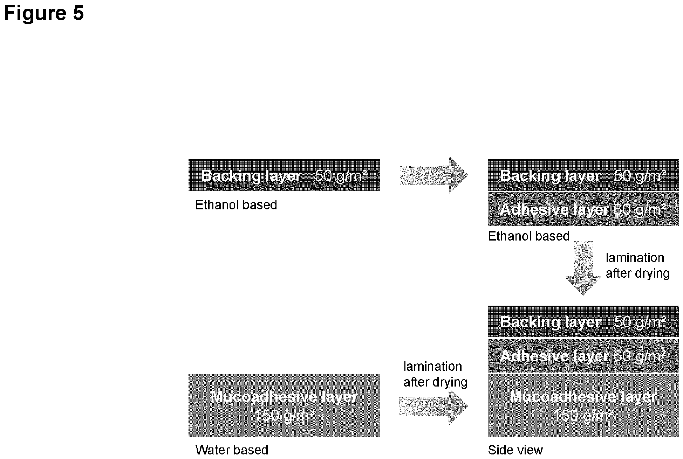

The composition of the MBF and the manufacturing process were optimized by including an intermediate layer, particularly an adhesive interlayer, between the mucoadhesive layer and backing layer. The combined backing layer and adhesive interlayer were dried before lamination on the dried mucoadhesive layer (Figure 5). Experiments using this revised manufacturing process and MBF composition showed that no Tacrolimus is detectable in the backing layer/adhesive interlayer, as examined using a dissolution test and HPLC. Accordingly, an adhesive interlayer prevents macrolide dissipation from the adhesive layer into the backing layer.

The features of the present invention disclosed in the specification, the claims, and/or in the accompanying figures may, both separately and in any combination thereof, be material for realizing the invention in various forms thereof.

## Claims

1. A mucoadhesive layer, preferably mucoadhesive buccal layer, comprising a non-nanoparticulate macrolide.

2. Mucoadhesive layer according to claim 1, wherein the non-nanoparticulate macrolide is selected from macrolide immune suppressants, macrolide antibiotics, and macrolide antifungals;
wherein, preferably,
- the macrolide immune suppressant is selected from tacrolimus, sirolimus, everolimus, and pimecrolimus;
- the macrolide antibiotic is selected from erythromycin, clarithromycin, azithromycin, roxithromycin, josamycin, spiramycin, telithromycin, tylosin, and fidaxomicin; and/or
- the macrolide antifungal is selected from polyenes, in particular nystatin, natamycin, and amphotericin B,
wherein, more preferably, said non-nanoparticulate macrolide is non-nanoparticulate tacrolimus or sirolimus, even more preferably non-nanoparticulate tacrolimus.

3. Mucoadhesive layer according to claim 1 or 2, wherein said non-nanoparticulate macrolide is present in said layer in the form of micronized macrolide, preferably micronized crystalline macrolide, and/or in a molecularly dissolved non-nanoparticulate form.

4. Mucoadhesive layer according to claim 3, wherein said micronized macrolide has an average particle size from 1 to 100 µm, preferably from 1.5 to 50 µm, more preferably from 2 to 25 µm.

5. Mucoadhesive layer according to any one of the foregoing claims, wherein the mucoadhesive layer comprises
- a mucoadhesive polymer, preferably a polymer selected from a) amphiphilic polymers and hydrophilic polymers, b) poly(methacrylates), c) crosslinked polyacrylic acid polymers, d) copolymers of methyl vinyl ether and maleic anhydride, e) polymers comprising polyvinyl acetate and/or polyvinylpyrrolidone, and combinations thereof;
wherein, preferably,
the amphiphilic polymers are selected from polyvinyl caprolactam-polyvinyl acetate-polyethylene glycol graft copolymers, polyoxyl castor oils and D-α-tocopherol-polyethylenglycol-succinate (TPGS), and/or
the poly(methacrylates) are selected from neutral protonizable poly(methacrylates) and cationic poly(methacrylates), preferably selected from copolymers of dialkylaminoethyl methacrylates and methacrylic acid ester(s), and copolymers of trialkylammonioethyl methacrylates and methacrylic acid ester(s), in particular copolymers of dimethylaminoethyl methacrylates and methacrylic acid esters, and copolymers of trimethylammonioethyl methacrylates and methacrylic acid esters;
and wherein the mucoadhesive layer further comprises:
- a cellulose derivative, preferably selected from hydroxypropyl cellulose (HPC), carboxymethyl cellulose (CMC), hydroxypropyl methylcellulose (HPMC), hydroxyethyl cellulose (HEC), methyl cellulose (MC), hydroxyethyl methyl cellulose (HEMC), ethyl cellulose (EC), and a combination thereof;
- a plasticizer; preferably selected from glycerol, polyethylene glycol, e.g. low molecular weight polyethylene glycol such as PEG 200 or PEG 400, propylene glycol, sorbitol, and tributylcitrate; more preferably glycerol;
- optionally a colorant, preferably TiO₂;
- and the non-nanoparticulate macrolide.

6. Mucoadhesive layer according to any one of claims 3 to 5, wherein said non-nanoparticulate macrolide is present in said layer in the molecularly dissolved non-nanoparticulate form;
and wherein said mucoadhesive polymer and/or said cellulose derivative is a polymer soluble in an organic solvent.

7. Mucoadhesive layer according to any one of the foregoing claims, wherein the mucoadhesive layer comprises
a) a mucoadhesive polymer, preferably an amphiphilic polymer; hydroxypropyl cellulose (HPC); carboxymethyl cellulose (CMC); a plasticizer, preferably glycerol; and optionally a colorant, preferably TiO₂;
preferably 10 wt% - 30 wt% of mucoadhesive polymer; 25 wt% - 35 wt% of hydroxypropyl cellulose (HPC); 10 wt% - 25 wt% of carboxymethyl cellulose (CMC); 10 wt% - 16 wt% of said plasticizer; and optionally 1 wt% - 4 wt% of said colorant; or
b) a mucoadhesive polymer, preferably selected from crosslinked polyacrylic acid polymers and copolymers of methyl vinyl ether and maleic anhydride; hydroxypropyl cellulose (HPC); ethyl cellulose (EC); a plasticizer, preferably glycerol; and optionally a colorant, preferably TiO₂;
preferably 2 wt% - 17 wt% of mucoadhesive polymer; 30 wt% - 70 wt% of hydroxypropyl cellulose (HPC); 5 wt% - 40 wt% of said ethyl cellulose (EC); 3 wt% - 20 wt% of said plasticizer; and optionally 1 wt% - 5 wt% of said colorant.

8. Mucoadhesive layer according to any one of the foregoing claims, wherein said macrolide is present in said mucoadhesive layer at a concentration of at least 2 wt%, preferably at least 3 wt%, e.g. 3.2 wt%; and/or in an amount in the range of from approximately 0.01 mg to approximately 10 mg, with reference to 1 cm² of said mucoadhesive layer, preferably in an amount in the range of from approximately 0.1 mg to approximately 5 mg, with reference to 1 cm² of said mucoadhesive layer, more preferably in the range of from approximately 1.5 mg to approximately 3.5 mg, with reference to 1 cm² of said mucoadhesive layer.

9. A mucoadhesive film, preferably mucoadhesive buccal film, comprising a mucoadhesive layer comprising a non-nanoparticulate macrolide, preferably a mucoadhesive layer as defined in any one of claims 1-8; and further comprising a backing layer, preferably a backing layer impermeable for the macrolide and/or impermeable for water.

10. Mucoadhesive film according to claim 9, wherein the backing layer comprises a plasticizer, preferably selected from glycerol, polyethylene glycol, e.g. low molecular weight polyethylene glycol such as PEG 200 or PEG 400, propylene glycol, sorbitol, and tributylcitrate, more preferably glycerol; and/or comprises a water-insoluble polymer and/or a cellulose derivative, e.g. hydroxyethyl cellulose, preferably a water-insoluble cellulose derivative, more preferably ethyl cellulose (EC).

11. Mucoadhesive film according to claim 9 or 10, wherein the mucoadhesive film further comprises an intermediate layer arranged between the mucoadhesive layer and the backing layer;
wherein, preferably, said intermediate layer facilitates adhesion between the mucoadhesive layer and the backing layer;
wherein, optionally, said intermediate layer comprises a plasticizer, preferably selected from glycerol, polyethylene glycol, e.g. low molecular weight polyethylene glycol such as PEG 200 or PEG 400, propylene glycol, sorbitol, and tributylcitrate, more preferably glycerol; and a polymer, preferably a polymer comprising polyvinyl acetate and/or polyvinylpyrrolidone, more preferably a vinylpyrrolidone-vinyl acetate copolymer.

12. Mucoadhesive film according to any one of claims 9-11,
wherein said mucoadhesive layer has an area weight in the range of from 70 g/m² to 180 g/m², preferably in the range of from 100 g/m² to 150 g/m²; and/or
wherein said backing layer has an area weight in the range of from 40 g/m² to 100 g/m², preferably in the range of from 40 g/m² to 60 g/m², e.g. 50 g/m²; and/or wherein, said intermediate layer, if present, has an area weight in the range of from 40 g/m² to 100 g/m², preferably in the range of from 40 g/m² to 70 g/m², e.g. 60 g/m².

13. A mucoadhesive film, preferably mucoadhesive buccal film, comprising a mucoadhesive layer, a backing layer, and an intermediate layer,
wherein said intermediate layer is arranged between the mucoadhesive layer and the backing layer, and wherein said mucoadhesive layer comprises a macrolide, preferably tacrolimus;
wherein, preferably, said intermediate layer facilitates adhesion between the mucoadhesive layer and the backing layer;
wherein, optionally, said intermediate layer comprises a plasticizer, preferably selected from glycerol, polyethylene glycol, e.g. low molecular weight polyethylene glycol such as PEG 200 or PEG 400, propylene glycol, sorbitol, and tributylcitrate, more preferably glycerol;
wherein, optionally, said intermediate layer comprises a polymer, preferably a polymer comprising polyvinyl acetate and/or polyvinylpyrrolidone, more preferably a vinylpyrrolidone-vinyl acetate copolymer.

14. Mucoadhesive layer according to any one of claims 1-8, or mucoadhesive film according to any one of claims 9-13, for use as a medicament.

15. Mucoadhesive layer according to any one of claims 1-8, or mucoadhesive film according to any one of claims 9-13, for use in preventing and/or treating transplant rejection, preferably solid organ transplant rejection such as liver, kidney or heart allograft rejection;
wherein, optionally, said use involves an attachment of said film or layer in the body cavity of a patient, preferably in a buccal cavity of a patient.

16. A method of preparing a mucoadhesive film, comprising
i) providing a micronized macrolide, preferably non-nanoparticulate micronized macrolide; and a polymer mixture, wherein said polymer mixture comprises
- a mucoadhesive polymer, preferably a polymer selected from a) amphiphilic polymers and hydrophilic polymers, b) poly(methacrylates), c) crosslinked polyacrylic acid polymers, d) copolymers of methyl vinyl ether and maleic anhydride, e) polymers comprising polyvinyl acetate and/or polyvinylpyrrolidone, and combinations thereof;
- a cellulose derivative, preferably selected from hydroxypropyl cellulose (HPC), carboxymethyl cellulose (CMC), hydroxypropyl methylcellulose (HPMC), hydroxyethyl cellulose (HEC), methyl cellulose (MC), hydroxyethyl methyl cellulose (HEMC), ethyl cellulose (EC), and a combination thereof;
- optionally, a plasticizer; preferably selected from glycerol, polyethylene glycol, e.g. low molecular weight polyethylene glycol such as PEG 200 or PEG 400, propylene glycol, sorbitol, and tributylcitrate; more preferably glycerol;
- optionally a colorant, preferably TiO₂;
ii) optionally, dispersing the micronized macrolide in a liquid medium to obtain a micronized macrolide dispersion and/or dissolving said micronized macrolide in an organic solvent to obtain a micronized macrolide solution;
iii) mixing the micronized macrolide, optionally micronized macrolide dispersion or micronized macrolide solution, with the polymer mixture to obtain a mucoadhesive layer composition;
iv) preparing a mucoadhesive layer using the mucoadhesive layer composition; optionally by coating the mucoadhesive layer composition on a surface, preferably on a process liner, e.g. by coating, hot melt extrusion, and/or casting;
v) optionally, drying said mucoadhesive layer, thereby obtaining the mucoadhesive film comprising said mucoadhesive layer.

17. The method according to claim 16, further comprising preparing a backing layer, comprising
a) providing a backing layer polymer mixture; optionally comprising a plasticizer, preferably glycerol; optionally comprising a water-insoluble polymer and/or a cellulose derivative, e.g. hydroxyethyl cellulose, preferably a water-insoluble cellulose derivative, more preferably ethyl cellulose;
b) preparing a backing layer using the backing layer polymer mixture, optionally on a surface of said mucoadhesive layer, e.g. by coating said polymer mixture on a surface of said mucoadhesive layer;
c) optionally, drying the backing layer.

18. The method according to claim 16 or 17, further comprising preparing an intermediate layer, comprising
1) providing an intermediate layer polymer mixture; optionally comprising a plasticizer, preferably glycerol, and a polymer, preferably a polymer comprising polyvinyl acetate and/or polyvinylpyrrolidone, more preferably a vinylpyrrolidone-vinyl acetate copolymer;
2) preparing an intermediate layer using the polymer mixture, optionally on a surface of said mucoadhesive layer, e.g. by coating said polymer mixture on a surface of said mucoadhesive layer;
3) optionally, drying the intermediate layer.
